# NEUE EUROPÄISCHE PATENTSCHRIFT

(11) **EP 1 232 392 B2**
(45) Veröffentlichungstag und Bekanntmachung des Hinweises auf die Entscheidung über den Einspruch: **02.04.2014**
(45) Hinweis auf die Patenterteilung: 02.04.2003
(21) Anmeldenummer: 00967861.6
(22) Anmeldetag: 12.10.2000
(51) Int. Cl.: G01N 33/569, C07K 16/12

(54) **VERBESSERTES VERFAHREN ZUM NACHWEIS VON SÄURE-RESISTENTEN BAKTERIEN DER GATTUNG HELICOBACTER IM STUHL**
IMPROVED METHOD FOR THE DETECTION OF ACID RESISTANT BACTERIA OF THE GENUS HELICOBACTER IN STOOL
PROCEDE DE DETECTION DE BACTERIES ACIDO-RESISTANTES DU GENRE HELICOBACTER DANS LES SELLES

(30) Priorität: 12.10.1999 EP 99120351; 16.03.2000 EP 00105592; 31.03.2000 EP 00107028; 10.05.2000 EP 00110110
(43) Veröffentlichungstag der Anmeldung: 21.08.2002
(60) Teilanmeldung: 03004839.1
(73) Patentinhaber: Oxoid (Ely) Limited, Angel Drove Ely CB7 4ET (GB)
(72) Erfinder: REITER, Christian, 85757 Karlsfeld (DE); CULLMANN, Gerhard, 81371 München (DE); HEPPNER, Petra, 82049 Pullach (DE); RINGEIS, Achim, 82166 Gräfelfing (DE); MÜLLER, Heidemarie, 81475 München (DE); HAINDL, Eva, 82349 Pentenried (DE)
(74) Vertreter: Eisenführ Speiser
(86) Internationale Anmeldenummer: PCT/EP2000/010058
(87) Internationale Veröffentlichungsnummer: WO 2001/027613

(56) Entgegenhaltungen:
- WO-A-00/26671
- WO-A-95/33482
- WO-A-97/34149
- WO-A-98/24885
- WO-A-99/44066
- WO-A-99/49889
- US-A- 5 200 344
- US-A- 5 607 863
- US-A- 5 932 430
- KRISHNAMURTHY P. ET AL.: "Helicobacter pylori containing only cytoplasmic urease is susceptible to acid." INFECTION AND IMMUNITY, Bd. 66, Nr. 11, November 1998 (1998-11), Seiten 5060-5066, XP002160658
- MAKRISTATHIS A ET AL.: "Two enzyme immunoassays and PCR for detection of Helicobacter pylori in stool specimens from pediatric patients before and after eradication tharapy." JOURNAL OF CLINICAL MICROBIOLOGY, Bd. 38, Nr. 10, Oktober 2000 (2000-10), Seiten 3710-3714, XP000983473

## Beschreibung

Die Erfindung betrifft ein Verfahren zum Nachweis einer Infektion eines Säugers mit einem Säure-resistenten Bakterium der Gattung Helicobacter, wobei man (a) eine Stuhlprobe des Säugers unter Verwendung (aa) eines Rezeptors unter Bedingungen inkubiert, die eine Komplexbildung eines Antigens aus dem Säure-resistenten Bakterium mit dem Rezeptor erlauben; oder (ab) zwei unterschiedliche Rezeptoren unter Bedingungen inkubiert, die eine Komplexbildung eines Antigens aus dem Säure-resistenten Bakterium mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Bakterium oder einem Extrakt oder Lysat davon oder einem Protein daraus oder einem Fragment davon oder einem synthetischen Peptid Antikörper produziert; und (b) die Bildung eines Antigen-Rezeptorkomplexes gemäß (a) nachweist, wobei das Antigen ein Antigen einer Katalase ist und der Nachweis der Bildung des einen Antigen-Rezeptor-Komplexes in Schritt (b) mittels ELISA, RIA, Western Blot oder eines immunochromatographischen Verfahrens erfolgt. Vorzugsweise ist das Bakterium *Helicobacter pylori,* oder *Helicobacter hepaticus*. Ferner betrifft die Erfindung diagnostische Zusammensetzungen und Testvorrichtungen, die vorgenannte Komponenten enthalten sowie diese enthaltende Verpackungen.

Der Nachweis der Infektion eines Säugerorganismus mit einem mikrobiellen Pathogen oder Parasiten kann heute auf verschiedene, invasive, semi-invasive oder nicht-invasive Arten geführt werden. Alle invasiven Methoden setzen eine Endoskopie und Biopsie voraus. Beim Einsatz dieser Techniken wird die körperliche Integrität des Untersuchten verletzt, z.B. bei der Entnahme einer Biopsie. Eine Entnahme von Biopsie ist aufwendig, verusacht hohe Kosten und bedeutet meist eine große Belastung für den Patienten. Da die Infektion mit bestimmten Mikroorganismen, beispielsweise mit *H. pylori* nicht über die gesamte Magenschleimhaut verteilt sein muß, ist durch Biopsie-Entnahme an einer nichtinfizierten Stelle ein falsch-negatives Ergebnis möglich. Ein weiterer Nachteil aller invasiven Methoden ist die Beeinflussung der Untersuchungsergebnisse durch frühere Behandlung mit Protonenpumpen-Hemmern, Wismut oder Antibiotika.

Semi-invasive oder nicht-invasive Diagnosetechniken stellen Veränderungen in Parametern fest, die ohne einen Eingriff in den Organismus gemessen werden können. Bevorzugt werden hierzu Körperflüssigkeiten und Ausscheidungen wie Serum, Atemluft, Urin, Speichel, Schweiß oder Stuhl, beprobt und analysiert. Bei direkten Methoden wird das Vorhandensein des Pathogens oder Parasiten, seiner Bestandteile oder deren Abbauprodukte durch Elektronenmikroskopie, optische Charakterisierung, Massenspektrometrie, Messung der radioaktiven Zerfallsprodukte oder spezifische enzymatische Reaktionen nachgewiesen. Oft sind diese Verfahren jedoch mit hohem apparativem Aufwand verbunden (z.B. Atemtest). Indirekte Verfahren greifen dagegen auf den Nachweis von Reaktionen des Wirtsorganismus auf das Pathogen oder den Parasiten zurück, z.B. das Vorhandensein von Antikörpern gegen Antigene des Pathogens im Serum oder im Speichel des Wirts.
Nachdem der Eingriff in den Organismus bei invasiven Techniken für den Organismus in den meisten Fällen belastend und häufig auch mit hohem apparativen Aufwand sowie einem gesundheitlichen Risiko verbunden ist, stellen nicht-invasive Techniken durch die vergleichsweise einfache Erfassung von Proben der oben beschriebenen Körperflüssigkeiten und Ausscheidungen die Methode der Wahl dar. Da weiterhin nicht jeder Wirt in gleicher Weise auf ein bestimmtes Pathogen oder einen bestimmten Parasiten reagiert, und die Reaktion des Wirts mit Verzögerung einsetzen und zudem auch nach Entfernung des Pathogens oder Parasiten aus dem Organismus persistieren kann, sind direkte Verfahren stets vorzuziehen. Idealerweise wird also eine Diagnose durch den nicht-invasiven, direkten Nachweis des Pathogens oder Parasiten in Körperflüssigkeiten oder Ausscheidungen geführt. Dies ermöglicht im Gegensatz zu indirekten Verfahren die Bestimmung des aktuellen Infektionsstatus.
Ein diagnostisches Verfahren sollte darüber hinaus auch auf weitere Gesichtspunkte hin optimiert sein: Hohe Reproduzierbarkeit, Sensitivität und Spezifität, garantierte Verfügbarkeit der zu verwendenden Materialien in konstanter Qualität, geringe Kosten bei Herstellung und Durchführung, und einfache Anwendung unabhängig von aufwendigen Apparaturen sind die hier zu berücksichtigenden Parameter.
Aus den oben genannten Gründen nehmen Verfahren basierend auf der hohen Selektivität und Bindungsaffinität bestimmter Substanzklassen (z.B. Antikörper, Rezeptoren, Lektine, Aptamere) für molekulare Strukturen, welche so gewählt werden können, daß sie für den jeweils zu bestimmenden Stoff hochspezifisch sind, in der medizinischen Diagnostik einen zunehmend breiten Raum ein. Insbesondere die Möglichkeit der Immobilisierung dieser Substanzen an Festkörperoberflächen sowie der Kopplung radioaktiver Nuklide, von Enzymen, die mit geeigneten Substraten Farbreaktionen auslösen oder farbiger Partikel mit der hochspezifischen Bindungsaffinität (z.B. im ELISA = Enzyme linked immunosorbent assay) führte zu der Entwicklung kostengünstiger, einfacher und wenig zeitaufwendiger Nachweisverfahren für körpereigene wie körperfremde Stoffe.
In den Anfangsphasen der Entwicklung dieser Nachweisverfahren fanden ausschließlich polyklonale Antikörper Verwendung. Diese haben jedoch einige, dem Fachmann wohlbekannte Nachteile, so vor allem begrenzte Verfügbarkeit und oft auch Kreuzreaktivitäten. Die Erarbeitung von Verfahren zur Herstellung monoklonaler Antikörper (Köhler & Milstein (1975)), die Fortschritte bei der Isolierung von Rezeptoren und deren gerichtete Expression in zellulären Wirtssystemen, die Entwicklung von Lektinen mit hoher Affinität für bestimmte Kohlenhydrate sowie die Entdeckung, daß einzelsträngige Nukleinsäure-Moleküle (Aptamere) molekulare Strukturen spezifisch binden können, konnten diese Nachteile großenteils beseitigen. Heute lassen sich mit vergleichsweise einfachen Methoden die Spezifität und Sensitivität von Nachweisverfahren optimieren.
Aufgrund der hohen Spezifität eignen sich derartige Verfahren besonders zum Nachweis einzelner, definierter Substanzen wie Haptene, Peptide oder Proteine, vorausgesetzt, das erkannte Strukturelement ist konstant innerhalb der zu untersuchenden Probenpopulation und spezifisch für die nachzuweisende Substanz. Sie sind zudem für eine Messung in Körperflüssigkeiten gut geeignet, und stellen damit eine naheliegende Option für den direkten Nachweis von Pathogenen in dieser Probenmatrix dar. Entsprechend sind im Stand der Technik Verfahren zur Diagnose z.B. von *Entamoeba histolytica* (Haque (1993), J. Infect. Dis. 167: 247-9), enterohemorrhagische *Escherichia coli* (EHEC; Park (1996), J. Clin. Microbiol. 34: 988-990), *Vibrio cholerae* (Hasan (1994), FEMS Microbiol. Lett. 120: 143-148), Torovirus-ähnliche Partikel (Koopmans (1993), J. Clin. Microbiol. 31: 2738-2744) oder *Taenia saginata* (Machnicka (1996), Appl. Parasitol. 37: 106-110) aus Stuhl beschrieben.
Den oben beschriebenen Pathogenen ist gemeinsam, daß sie im Darm ihres Wirts, in allen Fällen dem Menschen, lebens- und vermehrungsfähig sind. Sie besitzen also Mechanismen, die Ihnen das Überleben und die Vermehrung in Anwesenheit der im Darm aktiven Abbau- und Verdauungssysteme erlauben. Damit ist es wahrscheinlich, daß bei der Ausscheidung mit dem Stuhl eine hohe Anzahl intakter oder fast intakter Pathogene bzw. Parasiten abgehen. Mit Nachweisreagenzien, beispielsweise Antikörpern, welche die intakten Pathogene bzw. Parasiten erkennen, können diese im Stuhl oder in aufbereiteten Stuhlproben in der Regel leicht nachgewiesen werden.
Es gibt jedoch eine Anzahl von Pathogenen und Parasiten, die einerseits aufgrund der Beziehungen der von ihnen befallenen Gewebe (z.B. Lunge, Magen, Pankreas, Duodenum, Leber) zum Magen-Darm-Trakt im Stuhl auftreten können, andererseits aber im Darm selbst nicht lebens- und/oder vermehrungsfähig sind. Zu diesen Pathogenen und Parasiten gehören beispielsweise *Helicobacter pylori (H. pylori)* und *Helicobacter hepatis, Mycobacterium tuberculosis* und andere Mycobakterien, *Chlamydia pneumoniae, Legionella pneumophilae, Pneumocystis carinii*, und andere. Einige dieser Erreger können z.B. im Sputum nachgewiesen werden, jedoch ist beispielsweise der Nachweis von *Mycobacterium tuberculosis* im Sputum nur während eines kurzen Zeitraums möglich, und zwar nachdem sich eine den Erreger enthaltende Kaverne geöffnet hat. Ein Nachweis wird ferner dadurch erschwert, daß es nicht immer möglich ist, von einem zu Untersuchenden eine Sputumprobe zu erhalten. Dies trifft z.B bei Kleinkindern, verwirrten Patienten oder Tieren zu. Andere Pathogene wie beispielsweise *Legionella pneumophilae* lassen sich anhand von Antigenen, die über die Niere in den Urin gelangen spezifisch nachweisen. Dies gelingt jedoch nur, wenn die im Urin befindliche Menge für den Nachweis ausreicht. Der Nachweis im Stuhl wäre hierzu eine begrüßenswerte Alternative. Die Darmpassage ist bei diesen Organismen jedoch mit einem starken Angriff durch die Verdauungs- und Abbaumechanismen der Darmflora verbunden. Für den betrachteten Erreger spezifische molekulare Strukturen können dabei zerstört oder in ihrer Konzentration stark herabgesetzt werden.

Die Degradation der Erreger im Darm hat sich auch bei anderen Säure-resistenten Bakterien als Problem für einen sicheren Nachweis in Stuhlproben erwiesen. Die Zahl der Keime im Magen eines Infizierten ist im Vergleich zu anderen, sich im Darm ansiedelnden Bakterien, gering. Zudem müssen Keime und Keimbruchstücke nach Verlassen des Magens einen langen Weg durch den an Proteasen reichen Darm zurücklegen. Diese Umstände haben zur Folge, daß sich nur geringe Mengen intakter Proteine im Stuhl wiederfinden lassen, wobei man nicht davon ausgehen kann, daß immer die gleichen Fragmente bestimmter Proteine den Darmtrakt unbeschadet durchlaufen. Dies bedingt auch, daß die für einen ELISA-Test nötige Kombination zweier Epitope auf einem Antigen nicht mehr notwendigerweise wie im nativen Protein gegeben ist, und nah nebeneinander liegende Epitope die größte Wahrscheinlichkeit haben, in einem Nachweis, der zwei Epitope auf dem selben Molekül benötigt, ein positives Ergebnis zu zeigen. Idealerweise wird zum Nachweis nur ein Epitop auf dem selben Molekül benötigt. Die individuell unterschiedliche Verteilung von im Stuhl von Infizierten nachgewiesenen Antigenen weist zusätzlich auf individuelle Charakteristika in der Prozessierung der Antigene beim Darmdurchgang hin. Ein erster Ansatz, dieses Problem zu verringern, wurde durch den Offenbarungsgehalt der EP-A 0 806 667 bereitgestellt. In dieser Anmeldung wurde gezeigt, daß polyklonale Antikörper mit dem Lysat eines bestimmten *H. pylori*-Stammes induziert werden konnten, die eine breitere Variabilität von Stämmen aus verschiedenen geographischen Regionen erkennen. Allerdings geht aus dieser Anmeldung nicht hervor, welche Antigene durch das Serum erkannt werden. Angesichts der Tatsache, daß Immunseren trotz aller Standardisierungsbemühungen schwanken können, muß das in der o.g. Anmeldung entwickelte Verfahren für eine breite Anwendung als suboptimal betrachtet werden. Hinzu kommt, daß für die Bereitstellung der polyklonalen Seren immer wieder neue Tiere immunisiert werden müssen. Die entsprechenden Verfahren sind sowohl zeit- als auch kostenintensiv.

WO 98/24885 beschreibt die Herstellung monoclonaler Antikörper gegen ein Helicobacter pylori Antigen mit einem relativen Molekulargewicht von 16 ±2kDA und die Verwendung derartiger Antikörper zum Nachweis von H. pylori. Gemäß WO 98/24885 ist der Nachweis von H. pylori im Stuhl wie auch in der Mundhöhle besonders schwierig. Daher wird als notwendig angesehen, dass alternative Nachweisverfahren für dieses Bakterium entwickelt werden.

WO 00/26671, das als Stand der Technik im Simme von Art. 54(3) EPC betrachtet wird, beschreibt den Nachweis von säureresistenter Organismen, vorzugsweise H. pylori im Stuhl. Gemäß WO 00/26671 ist es notwendig, dass eine Stuhlprobe eines Säugers mit mindestens zwei verschiedenen monoclonalen Antikörpern, die gegen verschiedene Antigene gerichtet sind inkubiert wird, um eine aussagekräftige Diagnose zu erhalten.

Idealerweise wäre ein sicherer Nachweis der Infektion eines wie oben erweiterten Säure-resistenten pathogenen Organismus/Parasiten mit einem einzigen oder einer limitierten Anzahl für diesen pathogenen Organismus/Parasiten spezifischen Reagens/Reagentien möglich. Eine derartige Möglichkeit würde vor allem die Kosten für entsprechende Nachweisverfahren deutlich herabsetzen. Aufgabe der vorliegenden Erfindung war somit, ein entsprechendes Nachweisverfahren bzw. entsprechende Reagentien bereitzustellen.

Diese Aufgabe wird durch die in den Ansprüchen gekennzeichneten Ausführungsformen gelöst.

Somit betrifft die Erfindung ein Verfahren zum Nachweis einer Infektion eines Säugers mit einem Säure-resistenten Bakterium der Gattung Helicobacter, wobei man (a) eine Stuhlprobe des Säugers unter Verwendung (aa) eines Rezeptors unter Bedingungen inkubjert, die eine Komplexbildung eines Antigens aus dem Säureresistenten Bakterium mit dem Rezeptor erlauben; oder (ab) zwei unterschiedliche Rezeptoren unter Bedingungen inkubiert, die eine Komplexbildung eines Antigens aus dem Säure-resistenten Bakterium mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Bakterium oder einem Extrakt oder Lysat davon ooer einem Protein daraus oder einem Fragment davon oder einem synthetischen Peptid Antikörper produziert; und (b) die Bildung des einen Antigen-Rezeptorkomplexes gemäß (a) nachweist, wobei das Antigen ein Antigen einer Katalase ist und der Nachweis der Bildung des einen Antigen-Rezeptor-Komplexes in Schritt (b) mittels ELISA, RIA, Western Blot oder eines immunochromatographischen Verfahrens erfolgt.

Der Begriff "Säure-resistenter Mikroorganismus" im Sinne dieser Erfindung umfaßt jeden Mikroorganismus, der aufgrund seiner Eigenschaften/Anpassungsmechanismen an den Wirt den physikalischen und chemischen Einflüssen des Verdauungstrakts widersteht, so daß er durch einen vorzugsweise immunologischen Nachweis oder unter Verwendung von Aptameren detektierbar ist. Beispiele für derartige Säure-resistente Mikroorganismen sind *Helicobacter pylori, Helicobacter hepaticum, Mycobacterium tuberculosis, Mycobacterium pseudotuberculosis und Mycobacterium cansassii.*

Der Begriff "Stuhlprobe des Säugers" bedeutet im Sinne dieser Erfindung jede Stuhlprobe, die für das erfindungsgemäße Nachweisverfahren eingesetzt werden kann. Insbesondere fallen darunter Stuhlproben, die nach an sich bekannten Verfahren für diagnostische Tests aufbereitet worden sind. Die Aufbereitung erfolgt beispielsweise gemäß RIDASCREEN® Entamoeba Enzymimmunoassay (R-Biopharm GmbH, Darmstadt).

"Bedingungen, die eine Komplexbildung erlauben" sind vom Fachmann ohne weiteres einstellbar; s. auch Harlow und Lane, a.a.O., und sind beispielsweise physiologische Bedingungen.

Der Begriff "nach der Darmpassage eine Struktur aufweist, die der nativen Struktur entspricht" bedeutet im Sinne dieser Erfindung, daß das Epitop eines Antigens nach der Darmpassage von einem Rezeptor, beispielsweise einem monoklonalen Antikörper, Derivat oder Fragment davon erkannt wird, der/das gegen dasselbe Antigen/Epitop gewonnen wurde oder an dieses bindet, welches die Darmpassage nicht passiert hat. Mit anderen Worten, das Epitop/Antigen, das vom o.g. Rezeptor spezifisch gebunden wird, hat die Darmpassage hinsichtlich seiner Struktur unbeschadet oder im wesentlichen unbeschadet überstanden und ist nicht degradiert worden. Als Quelle für die native Struktur des Epitops/Antigens kann z.B. ein mit einer French-Press aufgeschlossener Bakterienextrakt, der mit üblichen Verfahren (vgl. z.B. Sambrook et al., "Molecular Cloning, A Laboratory Manual", 2. Auflage 1989, CSH Press, Cold Spring Harbor, USA) weiter aufgereinigt wurde, oder ein Bakterienlysat, das nach Standardverfahren weiter aufgereinigt wurde (z.B. Sambrook et al., a.a.O.), dienen.

Der Begriff "nach der Darmpassage eine Struktur aufweist, die der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert" bedeutet erfindungsgemäß, daß das vom Rezeptor erkannte Epitop einem Epitop entspricht, das vom Immunsystem eines Säugers, vorzugsweise eines Menschen, präsentiert wird. Die Mechanismen der Antigen-Präsentation sowie Mechanismen, die zur Prozessierung von Antigenen führen und die daraus resultierende Antikörpervielfalt sind im Stand der Technik bekannt und beispielsweise in Janeway und Travers, Immunologie, 2. Auflage 1997, Spektrum Akademischer Verlag GmbH, Heidelberg beschrieben. Diese Epitope können sich von den nativen Epitopen unterscheiden. Der Kontakt des Säugers mit den Mikroorganismen oder den Proteinen oder Fragmenten oder den synthetischen Peptiden kann durch natürliche Infektion (außer bei den synthetischen Peptiden) oder durch Immunisierung erfolgen. Für die Immunisierung können auch Extrakte, Lysate, synthetische Peptide etc. des Mikroorganismus/Proteins herangezogen werden. Geeignete Immunisierungsschemata sind im Stand der Technik bekannt und beispielsweise beschrieben in Harlow und Lane, a.a.O. Geeignete Antikörper können beispielsweise auch durch Immunisierung und/oder Screening auf Surrogate wie synthetische Peptide, rekombinant hergestellte Proteine, Extrakte, Lysate oder partiell verdaute Proteine gewonnen werden.

"Synthetische Peptide" umfassen solche Peptide, die mindestens ein Epitop des nativen oder durch den Darm passagierten Antigens aufweisen. Die Peptide können dabei dieselbe Primärstruktur wie das Antigen oder Fragmente davon aufweisen. Sie können jedoch auch eine andere Primärstruktur (primäre Aminosäuresequenz, z.B. konservative Austausche) aufweisen.

Der Begriff "spezifisch bindet" bedeutet erfindungsgemäß, daß der Rezeptor keine oder im wesentlichen keine Reaktivität mit anderen Epitopen in Proben nicht infizierter Säuger aufweist. Üblicherweise bindet der Rezeptor nur an das eine Epitop eines Antigens, das in der Stuhlprobe auftritt.

So kann in dieser Ausführungsform der Erfindung eine aufbereitete Stuhlprobe beispielsweise an eine Festphase gebunden werden und das infizierende Agens mit dem in markierter Form vorliegenden Rezeptor nachgewiesen werden. Sofern das nach der Darmpassage vorliegende Antigen (noch) in (homo) di- oder multimerer Form vorliegt, kann der gleiche Rezeptor sowohl als Fänger wie auch als Detektor eingesetzt werden.

Von Bedeutung ist für das erfindungsgemäße Verfahren ferner, daß für einen erfolgreichen Nachweis nur ein Epitop eines antigenen Proteins im wesentlichen konsistent nach der Darmpassage nachweisbar sein muß. Dieses Epitop kann auch mehrmals auf einem Homo-Dimer oder -multimer vorkommen. Die Wahrscheinlichkeit, daß dieses Epitop in nachweisbarer Form vorzufinden ist, ist aber wesentlich höher, als wenn ein Nachweistest auf mehr als einem nachzuweisenden Epitop aufbauen muß.

Schließlich bringt das erfindungsgemäße Verfahren, das nur einen Rezeptor benötigt, Kosten- und Standardisierungsvorteile mit sich.

Basierend auf dem erfindungsgemäßen überraschenden Befund, daß bestimmte Antigene aus den genannten Mikroorganismen nach der Darmpassage eine im wesentlichen konsistent nachweisbare Epitopstruktur aufweisen, muß auch eine zweite Ausführungsform als essentiell für die Erfindung gelten. Diese Ausführungsform beruht darauf, daß verschiedene Rezeptoren an verschiedene Epitope desselben Antigens binden. Der Begriff "im wesentlichen" bedeutet dabei, daß das Epitop/die Epitope und damit eine entsprechende Infektion mit dem Mikroorganismus bei mehr als 70%, vorzugsweise mindestens 75%, stärker bevorzugt mehr als 85%, besonders bevorzugt mehr als 90%, noch stärker bevorzugt mehr als 95% und am meisten bevorzugt mehr als 98% der Betroffenen erfaßt werden kann/können. Idealerweise werden Infektionen bei 100% der Betroffenen nachgewiesen.

Überraschenderweise wurde erfindungsgemäß gefunden, daß mit einem einzigen Rezeptor, der ein Epitop eines Antigens eines Säure-resistenten Bakterie der Gattung Helicobacter spezifisch bindet, oder zwei Rezeptoren, die zwei Epitope desselben Antigens spezifisch binden, eine relativ sichere Diagnose der Infektion mit diesen Bakterien/Pathogenen durchgeführt werden kann. Die Erfindung schließt Ausführungsformen mit ein, in denen weitere Epitope, die die vorgenannten Eigenschaften aufweisen, von weiteren Rezeptoren, z.B. von monoklonalen Antikörpern oder Fragmenten oder Derivaten davon erkannt werden. Letztere Ausführungsformen sind dazu geeignet, die Sicherheit bei der Stellung der Diagnose noch weiter zu erhöhen. Diese weiteren Rezeptoren können vorteilhafterweise Antikörper, Fragmente oder Derivate sein, die Urease, vorzugsweise β-Urease, das 26 kDa Protein oder Hsp 60, alle vorzugsweise aus *H*. *pylori,* spezifisch erkennen. Der Nachweis eines oder mehrerer dieser Proteine/Proteinfragmente kann im selben Test und in einem unabhängigen Test mit einem anderen Teil derselben Probe durchgeführt werden.

Die erfindungsgemäßen Ergebnisse sind vor allem deshalb überraschend, da der Stand der Technik hiervon weggelehrt hatte. So wurde beispielsweise bei *H. pylori* gefunden, daß Hauptantigene in ELISA-Tests nicht die gewünschte Spezifität und Sensitivität aufweisen; vgl. Newell et al., Serodiag. Immunother. Infect. Dis. 3 (1989), 1-6. Darüber hinaus lehrt die EP-A 0 806 667, daß ein sicherer Nachweis von *H. pylori*-Infektionen mit Rezeptoren wie monoklonalen Antikörpern aufgrund der genetischen Variabilität der *H. pylori*-Stämme nicht möglich sei.

Das erfindungsgemäße Verfahren ist gegenüber dem erwähnten Stand der Technik insbesondere deshalb von Vorteil, da mit lediglich einem Rezeptor eine relativ sichere Diagnose ermöglicht wird. Vorzugsweise werden für den Nachweis, beispielsweise im ELISA Pärchen von Rezeptoren, wie Antikörpern, Fragmenten oder Derivaten davon eingesetzt, wobei die beiden Rezeptoren des Pärchens dasselbe oder unterschiedliche Epitope auf demselben Antigen binden. Beispielsweise bildet *H. pylori*-Katalase multimere Strukturen aus mehreren gleichen Untereinheiten aus. Im ELISA oder anderen Assays können somit die gleichen Rezeptoren als Fängerrezeptoren wie auch als Detektionsrezeptoren eingesetzt werden. Ein weiterer Vorteil des erfindungsgemäßen Verfahrens ist seine Ausgestaltung als direktes und nicht-invasives Verfahren, was die einleitend genannten Annehmlichkeiten für den Patienten sowie die Zuverlässigkeit bei der Bestimmung des Krankheitsstadiums erhöht.

In einer anderen besonders bevorzugten Ausführungsform ist das Bakterium ein Bakterium der Spezies *Helicobacter pylori*, oder *Helicobacter hepaticum*

In einer weiteren bevorzugten Ausführungsform ist der Rezeptor/sind die Rezeptoren (ein) Antikörper, (ein) Fragment(e) oder (ein) Derivat(e) davon.

"Fragmente" oder "Derivate" von monoklonalen Antikörpern weisen im Sinne dieser Erfindung dieselbe Bindungsspezifität wie die monoklonalen Antikörper auf. Derartige Fragmente oder Derivate können nach üblichen Verfahren hergestellt werden; vgl. z.B. Harlow und Lane "Antibodies, A Laboratory Manual", CSH Press, Cold Spring Harbor, USA, 1988. Beispiele für Fragmente sind Fab-, F(ab')₂ oder Fv-Fragmente. Beispiele für Derivate sind scFv-Fragmente. Derivate können auch chemisch hergestellte Substanzen sein, die dieselben oder verbesserte Bindungseigenschaften wie die Antikörper aufweisen. Solche Substanzen können beispielsweise durch Peptidomimetics oder durch verschiedene Runden von Phage Display und nachfolgende Selektion auf verbesserte Bindungseigenschaften hergestellt werden.

Der Begriff "Antigen-Antikörperkomplex" im Sinne dieser Erfindung umfaßt nicht nur Komplexe, die das Antigen mit dem nativen Antikörper eingeht, sondern auch solche, die es mit dessen Fragmenten oder Derivaten eingeht.

Umfaßt von der Erfindung sind Ausführungsformen, bei denen nur monoklonale Antikörper oder Fragmente oder Derivate davon eingesetzt werden, wie auch Ausführungsformen, bei denen in einem Test unterschiedliche Arten von Nachweisreagenzien eingesetzt werden. So ist es möglich, daß ein erster monoklonaler Antikörper mit einem zweiten Antikörperderivat eingesetzt wird, um nur ein Beispiel zu nennen. Insofern bezeichnen die Begriffe "erste" und "zweite" das erste und zweite Nachweisreagens. Gemeint ist dabei nicht, daß immer zwei Antikörper, Derivate oder Fragmente davon eingesetzt werden.

Die Verwendung von monoklonalen Antikörpern, Fragmenten oder Derivaten davon gewährt einen leicht zu haltenden Standard bei der Zuverlässigkeit des Diagnoseverfahrens, was ein großer Vorteil im Vergleich zu bisher bekannten und für diesen Zweck eingeführten Diagnoseverfahren ist. Weiterhin entfällt das beispielsweise im Verfahren der EP-A 0 806 667 notwendige immer neue Immunisieren und nachfolgende Testen von Versuchstieren.

Gemäß der vorliegenden Erfindung ist das Antigen das Antigen einer Katalase, vorzugsweise aus *H. pylori*. Die Katalase hat den besonderen Vorteil, daß sie in allen bisher bekannten Säure-resistenten Bakterien nachgewiesen werden konnte. Erfindungsgemäß konnte als weiterer Vorteil ermittelt werden, daß die Katalase sehr resistent gegen die Verdauung im Darmtrakt ist, was den Nachweis signifikanter Mengen vereinfacht. Schließlich liegt die Katalase oder Fragmente davon auch nach der Darmpassage oft noch höhergeordneter Struktur, z.B. in tetramerer Form vor, was den Nachweis mit nur einem Rezeptortyp erleichtert.

Überraschenderweise wurde erfindungsgemäß gefunden, daß in einer Population von Säugern, insbesondere von menschlichen Patienten, deren Stuhl auf Infektionen mit Säure-resistenten Bakterien getestet wurde, im wesentlichen alle Mitglieder dieser Population konsistent wiederkehrende Katalase-Epitope im Stuhl aufwiesen, so daß mit hoher Wahrscheinlichkeit mit nur einem entsprechenden Rezeptor, vorzugsweise monoklonalen Antikörpern, Fragmenten oder Derivaten davon eine relativ sichere Diagnose gestellt werden kann. Insbesondere, da die Katalase eine tetramere antigene Struktur aufweist, kann diese Diagnose vorteilhafterweise beispielsweise im ELISA oder in ähnlich angeordneten Festsystem gestellt werden.

Besonders bevorzugt ist, daß die Katalase die Katalase von *H. pylori* ist.

In einer bevorzugten Ausführungsform wird für den Nachweis zusätzlich ein Gemisch von Rezeptoren eingesetzt, wobei das Gemisch von Rezeptoren als Fänger des Antigens fungiert, wenn der Rezeptor als Detektor des Antigens eingesetzt wird und das Gemisch als Detektor des Antigens fungiert, wenn der Rezeptor als Fänger des Antigens eingesetzt wird.
Diese Ausführungsform der Erfindung erlaubt eine besonders sichere Diagnose, insbesondere wenn das Antigen nicht in dimerer oder multimerer Konformation nach der Darmpassage vorliegt. Diese Ausführungsform erlaubt, daß nur einer der beiden in den meisten standardisierten immunologischen Nachweisverfahren eingesetzten Rezeptortypen ein monoklonaler Antikörper ist, während beispielsweise der zweite Rezeptortyp ein polyklonales Serum sein kann.

In einer besonders bevorzugten Ausführungsform ist das Gemisch von Rezeptoren ein polyklonales Antiserum.

In einer zusätzlich besonders bevorzugten Ausführungsform wurde das polyklonale Antiserum gegen ein Lysat des Bakteriums, vorzugsweise *H. pylori,* gewonnen.

In einer weiteren besonders bevorzugten Ausführungsform ist das Lysat ein Lysat mit angereichertem Antigen.

In einer anderen bevorzugten Ausführungsform ist das Lysat ein Lysat mit abgereichertem immundominantem Antigen.

Die vorgenannten beiden Ausführungsformen schließen auch ein, daß das Lysat ein Lysat mit angereichertem Antigen ist. Eine Art der Durchführung entsprechender Anreicherungs- bzw. Abreicherungsverfahren ist in den Beispielen näher beschrieben.

Gemäß einer weiteren besonders bevorzugten Ausführungsform wurde das polyklonale Antiserum gegen ein aufgereinigtes oder ein (semi)synthetisch hergestelltes Antigen gewonnen, das ein Katalase-, Antigen, vorzugsweise aus *H. pylori* ist.

Die Rezeptoren, vorzugsweise die monoklonalen Antikörper, Fragmente oder Derivate davon können erfindungsgemäß lineare oder Konformationsepitope erkennen und spezifisch binden. In einer weiteren bevorzugten Ausführungsform bindet mindestens einer der Rezeptoren ein Konformationsepitop.

In einer besonders bevorzugten Ausführungsform binden sämtliche Rezeptoren Konformationsepitope.

In einer besonders bevorzugten Ausführungsform weist die schwere Kette des ein Katalase-Epitop bindenden Antikörpers (HP 25.2m 2H10) mindestens eine der folgenden CDRs, vorzugsweise die CDR3 und weiter bevorzugt alle drei folgenden CDRs auf:

| | |
|---|---|
| CDR1: | NYWIH |
| CDR2: | YINPATGSTSYNQDFQD |
| CDR3: | EGYDGFDS |

In einer weiteren besonders bevorzugten Ausführungsform weist die die schwere Kette des ein Katalase-Epitop bindenden Antikörpers (HP 25.2m 2H10) kodierende DNA-Sequenz mindestens eine der folgenden CDRs, vorzugsweise die CDR3 und weiter bevorzugt alle drei folgenden CDRs auf:

| | |
|---|---|
| CDR1: | AACTACTGGA TTCAC |
| CDR2: | TACATTAATC CTGCCACTGG TTCCACTTCT TACAATCAGG ACTTTCAGGA C |
| CDR3: | GAGGGGTACG ACGGGTTTGA CTCC |

In einer anderen besonders bevorzugten Ausführungsform weist die leichte Kette des ein Katalase-Epitop bindenden Antikörpers (HP 25.2m 2H10) mindestens eine der folgenden CDRs, vorzugsweise die CDR3 und weiter bevorzugt alle drei folgenden CDRs auf:

| | |
|---|---|
| CDR1: | SASSSVNYMY |
| CDR2: | DTSKLAS |
| CDR3: | QQWSSNPYT |

Weiterhin weist in einer besonders bevorzugten Ausführungsform die die leichte Kette des ein Katalase-Epitop bindenden Antikörpers (HP 25.2m 2H10) kodierende DNA-Sequenz mindestens eine der folgenden CDRs, vorzugsweise die CDR3 und weiter bevorzugt alle drei folgenden CDRs auf:

| | |
|---|---|
| CDR1: | AGTGCCAGCT CAAGTGTAAA TTACATGTAC |
| CDR2: | GACACATCCA AATTGGCTTC T |
| CDR3: | CAGCAGTGGA GTAGTAATCC GTACACG |

In einer besonders bevorzugten Ausführungsform weist die schwere Kette des ein Katalase-Epitop bindenden Antikörpers [HP25.6m/1B5] mindestens eine der folgenden CDRs, vorzugsweise die CDR3 und weiter bevorzugt alle drei folgenden CDRs auf:

| | |
|---|---|
| CDR1: | DTYVH |
| CDR2: | KIDPANGKTKYDPIFQA |
| CDR3: | PIYYASSWFAY |

In einer weiteren besonders bevorzugten Ausführungsform weist die die schwere Kette des ein Katalase-Epitop bindenden Antikörpers (HP25.6m/1B5) kodierende DNA-Sequenz mindestens eine der folgenden CDRs, vorzugsweise die CDR3 und weiter bevorzugt alle drei folgenden CDRs auf:

| | |
|---|---|
| CDR1: | GACACCTATGTGCAC |
| CDR2: | AAGATTGATCCTGCGAATGGTAAAACTAAATATGACCCGATATTC CAGGCC |
| CDR3: | CCCATTTATTACGCTAGTTCCTGGTTTGCTTAC |

In einer anderen besonders bevorzugten Ausführungsform weist die leichte Kette des ein Katalase-Epitop bindenden Antikörpers (HP25.6m/1B5) mindestens eine der folgenden CDRs, vorzugsweise die CDR3 und weiter bevorzugt alle drei folgenden CDRs auf:

| | |
|---|---|
| CDR1: | KASQDVGTSVA |
| CDR2: | WTSTRHT |
| CDR3: | QQYSSSPT |

Weiterhin weist in einer besonders bevorzugten Ausführungsform die die leichte Kette des ein Katalase-Epitop bindenden Antikörpers (HP25.6m/1B5) kodierende DNA-Sequenz mindestens eine der folgenden CDRs, vorzugsweise die CDR3 und weiter bevorzugt alle drei folgenden CDRs auf:

| | |
|---|---|
| CDR1: | AAGGCCAGTCAGGATGTGGGTACTTCTGTTGCC |
| CDR2: | TGGACATCCACCCGGCACACT |
| CDR3: | CAGCAATATAGCAGCTCTCCCACG |

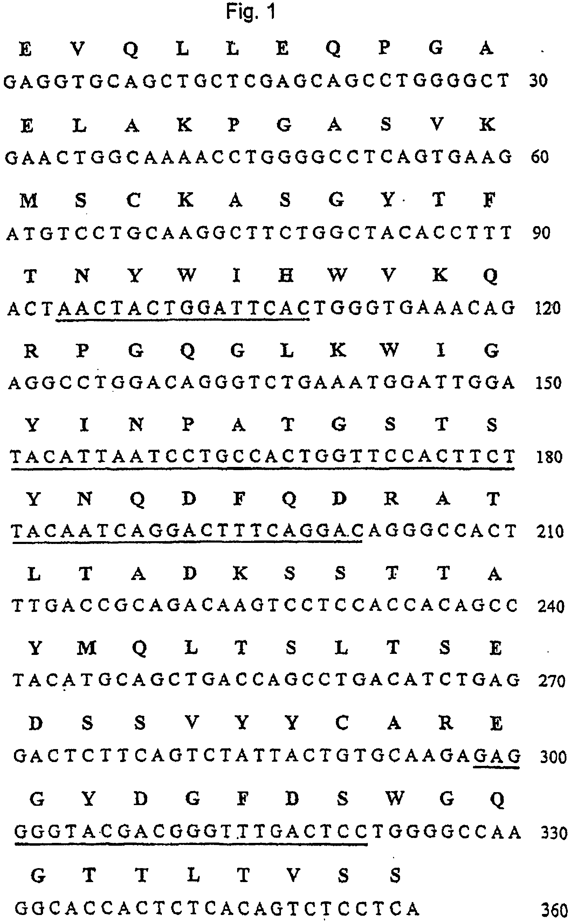

Besonders bevorzugt ist ferner, daß die schweren und leichten Ketten, die die vorstehend angegebenen CDRs aufweisen, gemeinsam in einem Antikörper, Fragment oder Derivat davon auftreten, der/das Katalase oder ein Fragment davon, vorzugsweise aus *H. pylori* spezifisch bindet. Die Erfindung umfaßt jedoch auch Ausführungsformen, in denen diese schweren oder leichten Ketten mit anderen leichten bzw. schweren Ketten kombiniert werden, wobei die Bindungseigenschaften im wesentlichen beibehalten oder verbessert werden können. Entsprechende Verfahren sind im Stand der Technik bekannt. Besonders bevorzugte Antikörper weisen in den variablen Regionen der leichten und schweren Ketten die in den Figuren 1 und 2 und den Figuren 3 und 4 dargestellten Aminosäuresequenzen auf bzw. werden die Regionen von den dort dargestellten DNA-Sequenzen kodiert. Die CDRs können nach im Stand der Technik bekannten Verfahren in verschiedene FRs ("framework regions") integriert werden.

In einer bevorzugten Ausführungsform werden mit der Stuhlprobe vor der Inkubation mit den Antikörpern folgende Schritte durchgeführt: Die Stuhlprobe wird 1:3 bis 1:25, vorzugsweise etwa 1:10, besonders bevorzugt 1:5 in einem Resuspendierungspuffer resuspendiert und daraufhin auf einem Vortexmixer gemischt. Ein beispielhafter Resuspendierungspuffer enthält, 150 mM PBS, 0,1% SDS. In einer bevorzugten Ausführungsform besteht der Resuspendierungspuffer aus 150 mM PBS, 0,5% tierischem Serum und 0,1% Detergens. Hierbei kann das tierische Serum aus Rind, Maus, Ratte oder Schwein gewonnen und das Detergens aus der Gruppe ionischer (besonders bevorzugt Tween 20), nicht-ionischer (besonders bevorzugt SDS) oder zwitterionischer Detergentien (besonders bevorzugt Chaps) ausgewählt werden.

Der hierin beschriebene Nachweis kann auch zur Detektion *von H. pylori* in Speichel oder Zahnplaque, eingesetzt werden. Zahnplaque wird nach entsprechend den im Stand der Technik bekannten Methoden gewonnen und kann wie Speichel in geeigneter Konzentration sowie Modifikation des Resuspendierungspuffers in den erfindungsgemäßen Nachweis eingesetzt werden.

Gemäß der vorliegenden Erfindung erfolgt der Nachweis der Bildung des einen Antigen-Rezeptorkomplexes in Schritt (b) mittels ELISA, RIA, Western Blot oder eines immunchromatographischen Verfahren. Derartige Verfahren sind an sich im Stand der Technik bekannt; vgl. Harlow und Lane, a.a.O.

In einer besonders bevorzugten Ausführungsform des erfindungsgemäßen Verfahrens wird im RIA oder im ELISA derselbe Rezeptor zur Bindung an die Festphase wie auch zum Nachweis des Epitops eingesetzt. Während der Fängerrezeptor in unmodifizierter Form an die Festphase, beispielsweise eine Mikrotiterplatte, gebunden werden kann, ist der zur Detektion eingesetzte Rezeptor gegebenenfalls mit einer Markierung versehen. Andererseits kann dieser Rezeptor ebenfalls nicht markiert sein und damit das bakterielle Epitop auch über einen dritten markierten Rezeptor nachgewiesen werden, wobei dieser Rezeptor vorzugsweise ein Antikörper, Fragment oder Derivat davon ist, der/das ein speziesspezifischer oder Ig-klassenspezifischer Antikörper sein kann. Markierungen von Antikörpern, beispielsweise mit radioaktiven oder fluoreszierenden Markern sind im Stand der Technik bekannt; vgl. Harlow und Lane a.a.O. Die vorstehend beschriebene Ausführungsform ist besonders günstig zum Nachweis der Katalase, die ggf. auch nach der Darmpassage noch als Tetramer vorliegt. Selbstverständlich können auch in dieser Ausführungsform Kombinationen von Antikörpern, Fragmenten und Derivaten eingesetzt werden, z.B. Kombinationen von Antikörpern etc., die an unterschiedliche Epitope desselben Antigens binden.

Unter einem Dreischritt-ELISA ist ein Verfahren zu verstehen, daß die Schritte Beschichtung der ELISA-Platte mit dem Fänger-Antikörper, Zugabe der Probe und Konjugatzugabe (wie beispielsweise markierter Detektorantikörper) sowie dazwischen geschaltete Waschschritte umfaßt. Der Einschritt-ELISA unterscheidet sich vom Dreischritt-ELISA dadurch, daß die Zugabe der Probe und die Zugabe des Konjugats auf eine mit dem Fänger-Antikörper vorbeschichtete ELISA-Platte in einem Schritt erfolgt.

In einer anderen bevorzugten Ausführungsform des erfindungsgemäßen Verfahrens ist der monoklonale Antikörper ein Maus-Antikörper.

Des weiteren sind in einer bevorzugten Ausführungsform die Rezeptoren an einen Träger fixiert.
Die Fixierung der Rezeptoren, vorzugsweise der Antikörper, Fragmente oder Derivate davon an einen Träger ist besonders vorteilhaft für die Durchführung von Routinechecks. Die Kombination Antikörper-Träger läßt sich ferner gut als Testbesteck oder in Kitform verpacken.

In einer besonders bevorzugten Ausführungsform ist das Trägermaterial ein poröses Trägermaterial.
In einer weiteren besonders bevorzugten Ausführungsform ist das Trägermaterial ein Teststreifen.

Zusätzlich besteht in einer bevorzugten Ausführungsform das Trägermaterial aus Zellulose oder einem Zellulosederivat.

Der Säuger, dessen Stuhl etc. mit dem erfindungsgemäßen Verfahren untersucht werden kann, kann ein Tier, beispielsweise ein Haustier wie eine Katze oder ein Hund, ein Nutztier, z.B. ein Schwein oder ein sonstiges Tier wie eine Maus, ein Tiger, ein Gerbil oder ein Frettchen sein.

In einer bevorzugten Ausführungsform ist der Säuger ein Mensch.

In einer weiteren bevorzugten Ausführungsform ist das erfindungsgemäße Verfahren ein automatisiertes Verfahren. Ein automatisiertes Verfahren kann beispielsweise mittels eines Roboters durchgeführt werden, wobei der Roboter einen Teil der oder sämtliche Verfahrensschritte durchführt. Entsprechende Roboter sind im Stand der Technik bekannt.

Darüber hinaus betrifft die Erfindung einen ein Katalase-Epitop bindenden monoklonalen Antikörper, ein Fragment oder Derivat davon, der/das eine V-Region aufweist, die eine Kombination der vorstehend dargestellten CDRs aufweist oder der von einem der vorstehend dargestellten Hybridomen produziert wird.

Bevorzugt ist dabei ein monoklonaler Antikörper, Fragment oder Derivat davon, der/das mindestens eine der in den Figuren 1 und 2, 3 und 4 dargestellten V-Regionen aufweist. Vorzugsweise weist dieser Antikörper zwei der in den Figuren 1 und 2, 3 und 4 dargestellten V-Regionen auf. Auch ist bevorzugt, daß diese V-Regionen von den in den Figuren 1 und 2, 3 und 4 dargestellten DNA-Sequenzen kodiert werden.

In einer besonders bevorzugten Ausführungsform der Erfindung ist der monoklonale Antikörper, das Fragment oder Derivat davon ein Maus-Antikörper oder ein Fragment oder Derivat davon oder ein chimärer, vorzugsweise ein humanisierter Antikörper oder ein Fragment oder Derivat davon. Das Derivat kann auch ein Fusionsprotein sein. Weiter bevorzugt ist, daß der Antikörper markiert ist, beispielsweise mit einem Kolloid, mit einer radioaktiven, fluoreszierenden, phosphoreszierenden oder chemiluminiszierenden Markierung.
Die Herstellung von chimärisierten humanisierten und humanen Antikörpern und der anderen Derivate ist im Stand der Technik wohlbekannt (z.B. Vaughan et al., 1998; Orlandi et al., 1989, Harlow und Lane, a.a.O.).

Die vorliegende Erfindung betrifft darüber hinaus die Vervendung einer diagnostischen Zusammensetzung enthaltend einen Rezeptor der ein Antigen einer Helicobacter Katalase spezifisch bindet, bevorzugt einen monoklonalen Antikörper, Fragmente oder Derivate davon wie oben stehend definiert, gegebenenfalls fixiert an ein Trägermaterial.

Des weiteren betrifft die vorliegende Erfindung eine Testvorrichtung zum Nachweis mindestens eines wie oben stehend definierten Epitops, umfassend (a) einen Rezeptor der ein Antigen einer Helicobacter Katalase spezifisch bindet, der vorzugsweise ein monoklonaler Antikörper, Fragmente oder Derivate davon ist wie oben stehend definiert, fixiert an ein Trägermaterial; und (b) eine Vorrichtung zur Aufbereitung und Analyse von Stuhlproben.

Die Erfindung hat ferner zum Gegenstand eine Testvorrichtung enthaltend (a) einen Rezeptor, vorzugsweise einen monoklonalen Antikörper, Fragmente oder Derivate davon wie oben stehend definiert, wobei der Rezeptor konjugiert ist mit kolloidalem Gold, Latexpartikeln oder anderen farbgebenden Partikeln, deren Größe typischerweise im Bereich zwischen 5nm und 100nm, vorzugsweise zwischen 20nm und 60nm liegt (besonders bevorzugt ist eine Partikelgröße für Gold zwischen 40nm und 60nm und für Latex zwischen 200nm und 500nm); und (b) eine Vorrichtung zur Aufbereitung und Analyse von Stuhlproben.

Darüber hinaus betrifft die vorliegende Erfindung einen Kit enthaltend (a) einen Rezeptor der vorzugsweise ein monoklonaler Antikörper, Fragmente oder Derivate davon wie oben stehend definiert ist, gegebenenfalls fixiert an ein Trägermaterial; gegebenenfalls ferner (b) eine Vorrichtung zur Aufbereitung und Analyse von Stuhlproben.

Schließlich betrifft die Erfindung eine Packung enthaltend die erfindungsgemäße diagnostische Zusammensetzung, die erfindungsgemäße Testvorrichtung oder den erfindungsgemäßen Kit.
Die Bestandteile der erfindungsgemäßen diagnostischen Zusammensetzung, der erfindungsgemäßen Testvorrichtung und/oder des erfindungsgemäßen Kits können in Behältern, wie beispielsweise Fläschchen oder Röhrchen, gegebenenfalls in Puffern und/oder Lösungen verpackt sein. Unter Umständen können eine oder mehrere der Bestandteile in ein- und demselben Behälter verpackt sein.

### Die Figuren zeigen:

Fig. 1: Klonierte DNA-Sequenz, die für die V-Region der schweren Kette eines für Katalase spezifischen monoklonalen Antikörpers (HP 25.2m 2H10) kodiert. Die kodierte Aminosäuresequenz ist im Einbuchstaben-Code dargestellt. Die nach Kabat et al. bestimmten CDR-Regionen 1-3 sind durch Unterstreichung hervorgehoben.

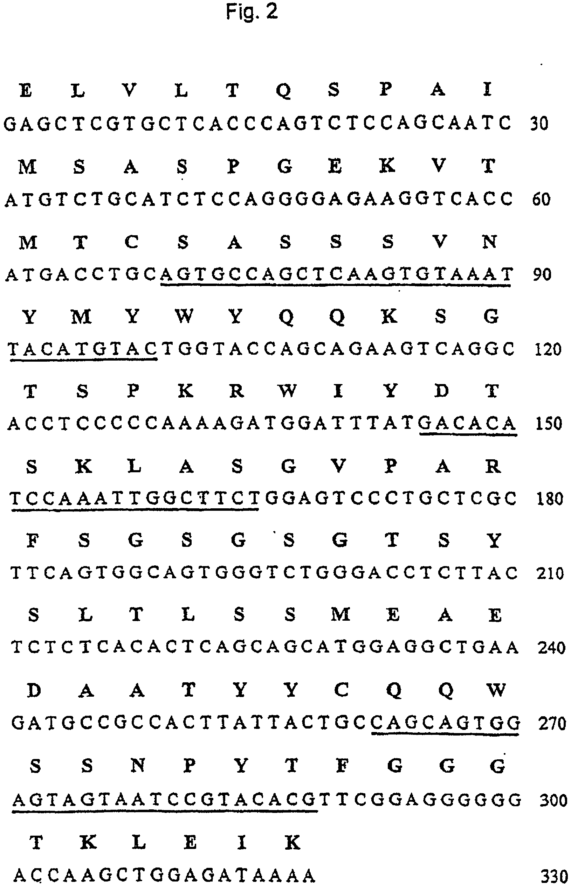

Fig. 2: Klonierte DNA-Sequenz, die für die V-Region der leichten Kette eines für Katalase spezifischen monoklonalen Antikörpers (HP 25.2m 2H10) kodlert. Die kodierte Aminosäuresequenz ist im Einbuchstaben-Code dargestellt. Die nach Kabat et al. bestimmten CDR-Regionen 1-3 sind durch Unterstreichung hervorgehoben.

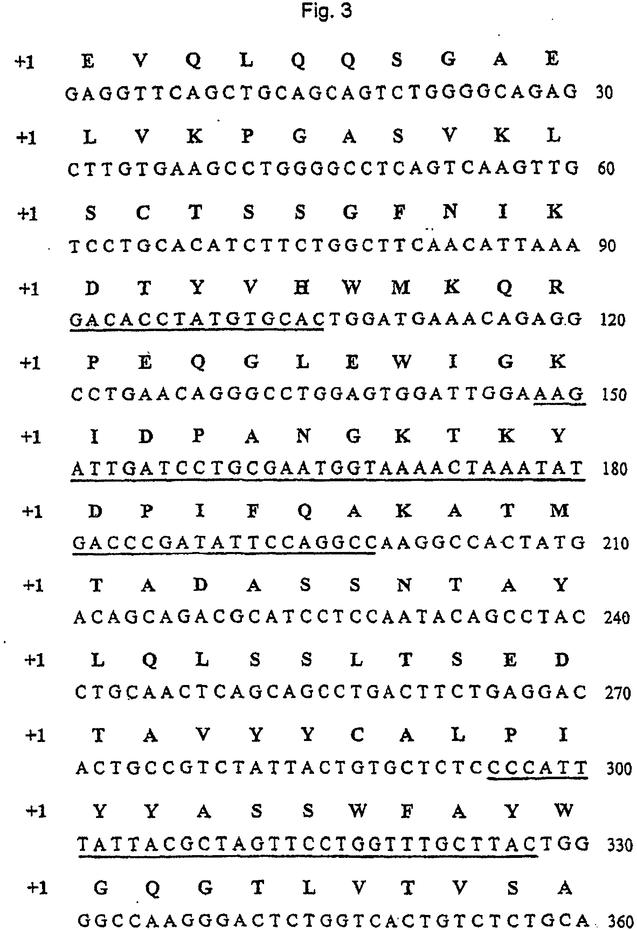

Fig. 3: Klonierte DNA-Sequenz, die für die V-Region der schweren Kette eines für Katalase spezifischen monoklonalen Antikörpers (HP25.6m/1B5) kodiert. Die kodierte Aminosäuresequenz ist ebenfalls dargestellt. Die nach Kabat et al. bestimmten CDR-Regionen 1-3 sind durch Unterstreichung hervorgehoben.

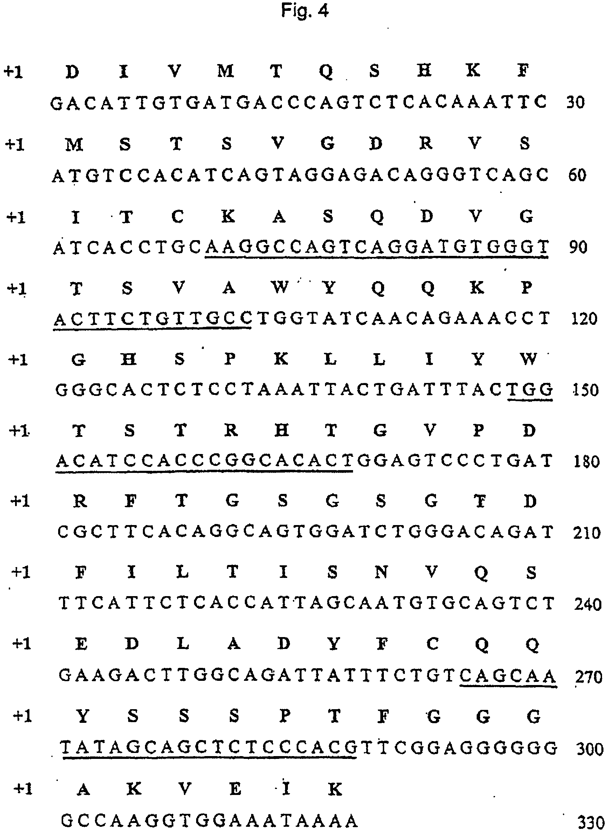

Fig. 4: Klonierte DNA-Sequenz, die für die V-Region der leichten Kette eines für Katalase spezifischen monoklonalen Antikörpers (HP25.6m/1B5) kodiert. Die kodierte Aminosäuresequenz ist ebenfalls dargestellt. Die nach Kabat et al. bestimmten CDR-Regionen 1-3 sind durch Unterstreichung hervorgehoben.

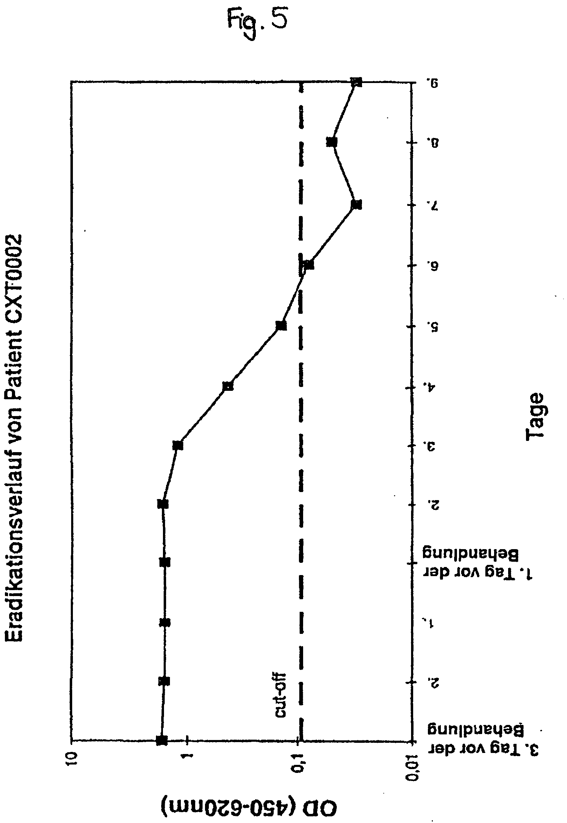

Fig. 5: Verlauf einer Eradikationsbehandlung eines *H. pylori*-positiven Patienten nach Einnahme von Omeprazol, Metronidazol und Clarithromycin.

Die Beispiele erläutern die Erfindung.

### Beispiel 1: Isolierung von H. pylori Antigenen

### 1.1 Kultivierung von H. pylori

*H. pylori* (Stamm NCTC 11637) wurde in Petri-Schalen auf Wilkins-chalkern Agar unter Zusatz von 10% Pferdeblut sowie Amphotericin B, Vancomycin und Cefsoludin (Sigma Chemicals) ausgestrichen und 1-2 Tage unter mikroaerophiler Atmosphäre (Anaerocult GasPAk, Merck) bei 37ºC inkubiert. Der Inhalt von 2 Schalen wurde in 350ml BHIB-Medium unter Antibiotika-Zusatz wie oben in einer 1l Flasche (Schott) suspendiert, das Medium für 4-8 min mit einem Gasgemisch aus 10% CO₂, 5% O₂, 85% N₂ begast und die Flasche verschlossen. Die Kultur wurde 2 Tage bei 37 ºC auf einem Rundschüttler geschüttelt. Der Inhalt der Flasche wurde anschließend steril in eine 10l Flasche überführt und mit 4,7l BHIB-Medium aufgefüllt. Die Flasche wurde dann weitere 2 Tage bei 37ºC auf einem Rundschüttler inkubiert. Das gesamte Volumen wurde daraufhin bei 5000g für 15min zentrifugiert, Überstand dekantiert und das Bakterienpellet gewogen. Zur Lagerung wurde das Pellet in einer physiologischen Kochsalzlösung unter Zusatz von 15% Glycerin im Verhältnis 2:1 (w/v) resuspendiert und bei -80ºC eingefroren. Um die Identität der kultivierten Bakterien zu überprüfen wurde eine mikroskopische Inspektion der Bakterien sowie Tests auf Urease-, Oxidase und Katalase-Aktivität durchgeführt.

### Beispiel 2: Präparation von H. pylori Antigenen

### Präparation von H. pylori-Lysat

*H. pylori* Bakterienpellet (Beispiel 1) wurde 1:10 mit PBS, pH 7,5 versetzt und auf Eis resuspendiert. Die Bakterienzellen wurden auf Eis mit der kleinen Sonde eines Ultraschallgerätes (Sonifier, Branson), bei 25 - 30 % Intensität 10 x 60s mit jeweils 60s Pause beschallt. Die aufgeschlossenen Bakterienzellen wurden 2 x 20 min, bei 4°C und 10.000 UpM zentrifugiert (Sorvall, SS34). Der Überstand wurde als Antigenpräparation für die Produktion von polyklonalen Antiseren verwendet.

### Präparation von H. pylori Katalase

Gefrorenes Bakterienpellet wurde im Verhältnis 1:2 (w / v) mit Aufschlußpuffer (20 mM Tris HCI pH 7.0, 1mM EDTA, 1 mM Phenyl-Methyl-Sulfonyl-Flourid (PMSF), 0,05% Natriumazid und 10% (v / v) Isobutanol) versetzt und bei Raumtemperatur (RT) auf einem Über-Kopf-Mischer bis zum vollständigen Auftauen und zusätzlich weitere ca. 15 min geschüttelt. Nach Zentrifugation bei 20.000g, 4°C für 20 min, wurde der Überstand abdekantiert und über ein 0,45 µm Filter filtriert.

Der klare Überstand wurde im Verhältnis 1:3 mit Puffer A (20 mM Tris HCI, pH 7.0, 1mM EDTA, 1 mM PMSF, 0,05% Natriumazid) verdünnt und auf eine mit Puffer A äquilibrierte SourceQ-Säule (16/10) (Pharmacia) überführt. Der Durchlauf von der SourceQ-Säule enthielt das Enzym Katalase und war frei von *H. pylori* Hauptantigenen wie Urease, HSP60 und Alkylhydroperoxid-Reduktase.
Zur Isolierung der Katalase wurde der Durchlauf von der SourceQ-Säule einer Molekularsieb-Chromatographie (Superdex 200) (16/60) unterzogen. Die Katalase wurde dabei zusammen mit einem andern ca. 150 kDa großen Protein (Neutrophil Activating Protein, NAP) in etwa gleichen Anteilen isoliert.
In höherer Reinheit wurde Katalase erhalten, wenn der Durchlauf von der SourceQ-Säule mit einer 2 M Natriumacetat-Lösung, pH 4.9 auf 40 mM Natriumacetat gebracht und auf eine SourceS-Säule (8/28) überführt wurde. Nach einem Waschschritt mit Puffer A zur Entfernung nicht gebundener Proteine wurde die Katalase mit Puffer B (40 mM Natriumacetat, 1 M NaCl, pH 4.9) unter Verwendung eines linearen NaCI-Gradienten (Puffer A plus 0% bis 100% Puffer B) eluiert. Katalase eluiert bei ca. 370 mM NaCl.

### Beispiel 3: Charakterisierung der Katalase:

Das gereinigte Protein wies unter reduzierenden Bedingungen im SDS-PAGE ein Molekulargewicht von ca. 58 kDa und eine Reinheit von ≥ 90% auf.

Zur Identifizierung des isolierten Proteins wurde eine Mikrosequenzierung durchgeführt. Das Protein wurde im SDS-PAGE Gel mit LysC Protease gespalten. Das extrahierte Proteingemisch wurde über RP-HPLC aufgetrennt. Die Sequenzanalyse des LysC Peptides ergab folgende Aminosäure-Sequenz:

### ERLHDTIGESLAHVTHK

Diese Sequenz ist identisch mit dem entsprechenden LysC-Peptid aus *H. pylori* Katalase (Manos J. et al. (1998) Helicobacter 3 (1), 28-38; Genbank Accession No AAC16068.1)

### Beispiel 4: Herstellung polyklonaler und monoklonaler Antikörper (pAk; mAk)

### Herstellung polyklonaler Antiseren:

Polyklonale Antiseren gegen *H. pylori-*Lysat, *H. pylori*-Lysat mit abgereicherten Hauptantigenen wie beispielsweise Urease, HSP60 und Alkylhydroperoxid-Reduktase (siehe Beispiel 2: Isolierung und Reinigung), *H. pylori*-Lysat mit angereicherter Katalase (beispielsweise durch Zufügen von Katalase zum Lysat) sowie polyklonale Antiseren gegen gereinigte Katalase können durch Immunisierung eines ausgewählten Säugetieres (z.B. Maus, Kaninchen, Ziege, etc.) mit den entsprechenden Katalase-Epitope enthaltenden immunogenen Präparationen erhalten werden.

Die Antikörper können mittels Protein A Affinitäts-Chromatographie aus Seren gereinigt und als Fang-Antikörper im Sandwich-ELISA (siehe Beispiel 9) zur Beurteilung der Eignung monoklonaler Antikörper für die Antigen-Detektion in Patientenstuhl eingesetzt werden.

Polyklonale Kaninchen-Antiseren wurden von pab Productions (Herbertshausen) aus *H. pylori*-Lysat hergestellt. Aus diesen Antiseren wurden mittels Protein A Affinitäts-Chromatographie polyklonale Antikörper aufgereinigt und als Fang-Antikörper im Sandwich-ELISA (siehe Beispiel 9) zur Beurteilung der Eignung monoklonaler Antikörper für die Antigen-Detektion in Patientenstuhl verwendet.

### Herstellung monoklonaler Antikörper:

Die Herstellung monoklonaler Antikörper erfolgt nach dem Fachmann bekannten Methoden (Harlow & Lane, 1988; Peters & Baumgarten, 1990).

### Immunisierung

Aus *H. pylori*-Lysat hergestellte Antigenpräparationen (siehe Beispiel 2), wurden zur Immunisierung von Mäusen (BALB/c x C57/Black, F1-Generation, 8-12 Wochen alt) verwendet. Als Grundimmunisierung wurden 50 µg Antigen 1:1 mit komplettem Freundschem Adjuvans (Difco), emulgiert und intraperitoneal injiziert (200 µl/Maus). Bei 4-monatlichen Auffrischungen erhielten die Mäuse jeweils 25 µg Antigen mit inkomplettem Freundschem Adjuvans. Aus retroorbital entnommenem Blut wurde Antiserum als Positivkontrolle im ELISA (siehe Fusionsscreening) gewonnen.

### Fusion

Zwei Tage nach der letzten Immunisierung wurden den Mäusen die Milzen entnommen und die Milzzellen mit den Myelomzellen P3x63Ag8.653 (ATCC CRL-1580; Kearney et al., 1979) im Verhältnis 5:1 mit Polyethylenglykol 4000 fusioniert. Die fusionierten Zellen wurden in HAT-Medium (Klonierungsmedium (= RPMI 1640 Medium, 20% FCS, 200 U/ml rhlL-6) mit Hypoxanthin-Aminopterin-Thymidin-Supplement (100x Konzentrat, Sigma)) suspendiert und mit einer Zelldichte von 2-6x10⁴ Zellen/Napf in 96-Napf-Mikrotiterplatten ausplattiert. Die Kultivierung der Hybridome erfolgte bei 37°C, 5% CO₂ und 95% relativer Luftfeuchtigkeit.

### Fusionsscreening mittels direktem ELISA

Das Screening der antikörperhaltigen Kulturüberstände aus bewachsenen Näpfen (ca. 10 Tage nach der Fusion) erfolgte im direkten ELISA auf 96-Napf Mikrotiterplatten (MaxiSorb, Nunc):
Die ELISA-Platten wurden mit 2 µg/ml Immunisierungsantigen in Carbonatpuffer, pH 9,6 beschichtet (100 µl/Napf, über Nacht, 5°C). Die Beschichtungslösung wurde abgesaugt und noch freie Bindungstellen mit 2% Magermilchpulver in PBS (w / v) geblockt (200 µl/Napf 1h, Raumtemperatur). Nach zweimaligem Waschen der Platte mit PBS pH 7,3 mit 0,025% Tween 20 (v / v) wurden die Kulturüberstände der Primärklone unverdünnt in die Näpfe pipettiert (100 µl/Napf) und die Platten 1-2 Stunden bei Raumtemperatur inkubiert. Als Positivkontrolle wurde Antiserum, als Negativkontrolle Medium verwendet. Nach erneutem Waschen erfolgte die Detektion der gebundenen Antikörper mit einem Peroxidase-markierten Sekundärantikörper (Kaninchen-anti-Maus Ig-POD (DAKO) in PBS mit 0,1% Rinderserumalbumin, 20min, Raumtemperatur). Die Peroxidase setzt im nachfolgenden Schritt das farblose Substrat Tetramethylbenzidin (TMB, Sigma) zu einem farbigen Komplex um. Nach viermaligem Waschen und Ausklopfen der Platte wurde Substratlösung (K-Blue, Neogen oder Zitronensäurepuffer, pH 4,5 mit TMB + H₂O₂) zugegeben und die Reaktion nach 10min durch Zugabe von 1 N Schwefelsäure abgestoppt. Kulturüberstände von Klonen, die antigenspezifische Antikörper produzieren, zeigten eine deutliche Färbung, gegenüber den farblosen negativen Kulturüberständen.

### Etablierung und Kultivierung der Hybridome

Positive Klone wurden zweimal nach dem Prinzip der Grenzverdünnung rekloniert, um Monoklone zu erhalten (Coller & Coller, 1983). Die erste Reklonierung erfolgte in Klonierungsmedium mit Hypoxanthin-Thymidin-Supplement (100x Konzentrat, Sigma), die zweite in Klonierungsmedium. Die Reklone wurden wiederum mittels direktem ELISA auf Antigenspezifität hin überprüft. Der Endklon wurde schließlich in Flachflaschen an Produktionsmedium (RPMI 1640 Medium mit 5% IgGreduziertem FCS) adaptiert. Die Zellen wurden kryokonserviert und Kulturüberstand für die Antikörperreinigung produziert.

### Beispiel 5: Charakterisierung der Antikörper aus Kulturüberstand

Aus einem Repertoire von 30 spezifischen (gegen das Immunisierungs-Antigen Antikörper-produzierend) Klonen wurden 10 anhand guter Reaktivität auf Stuhlproben *H*. *pylori*-infizierter Patienten im Sandwich-ELISA ausgewählt (sieheTabelle 2).

### Isotypbestimmung

Bei den etablierten Klonen wurde im Kulturüberstand eine Isotypbestimmung des monoklonalen Antikörpers mit dem Isotyping Kit IsoStrip (Roche Diagnostics) durchgeführt. Dies ergab 8 Klone des Typs IgG1 und einen Klon IgG2a (siehe Tabelle 3).

### Westernblot

Die Kulturüberstände wurden im Westernblot auf die Fähigkeit überprüft, das Immunisierungs-Antigen spezifisch zu erkennen. Pro Gel wurden 15 µg gereinigtes Antigen in reduzierendem Probenpuffer (Laemmli, 1970) gekocht und auf ein 12%iges SDS-Poplyacrylamid-Minigel (8,6cm x 7,7cm x 0,1cm, Biometra) aufgetragen. Nach elektrophoretischer Auftrennung bei 25-30 mA wurden die Proteine (Antigen) mittels Semidry-Blot-Verfahren auf einer Nitrozellulose-Membran immobilisiert.
Die Membran wurde mit 2% Magermilchpulver in PBS geblockt (30min, Raumtemperatur) und dreimal 5min. mit TBS/Tween 20 (0,2%) gewaschen. Für den folgenden Inkubationsschritt wurde die Membran in eine Accutran Cross-Blot-Screening-Einheit (Schleicher und Schüll) eingespannt, unter Verwendung einer Gitterplatte mit 34 Querkanälen. In jede der entstandenen Spuren wurden 250µl TBS/Tween 20 vorgelegt und je 250µl der zu testenden Hybridomakulturüberstände zugegeben. Die Inkubation erfolgte 2h bei Raumtemperatur unter Schütteln.

Nach dreimaligem Waschen TBS/Tween 20 wurde die Membran 1h mit dem PODkonjugierten Sekundärantikörper (Kaninchen-anti-Maus Ig-POD, DAKO) inkubiert. Die Membran wurde dreimal gewaschen und der Immunkomplex durch Zugabe der 3,3-Diaminobenzidine-Substratlösung (DAB, Sigma) visualisiert. Die antikörperbindenden Proteinbanden wurden anschließend durch ein unlösliches Peroxidasesubstrat sichtbar gemacht.
6 Hybridomakulturüberstände zeigten eine der Katalase entsprechende Bande (58 kDa), 3 waren im Westernblot negativ, zeigen jedoch eine positive Reaktion mit nativem Antigen im ELISA. Wahrscheinlich erkennen sie ein Konformationsepitop. Die Ergebnisse sind in Tabelle 3 zusammengefaßt.

### Beispiel 6: Reinigung von mAk aus Hybridomakulturüberständen

Die Reinigung von mAk aus serumfreien Hybridomakulturüberständen erfolgt mittels einer modifizierten Protein-G Affinitätschromatographie (Pharmacia Biotech, 1994).
Die filtrierten (0,45µm) Kulturüberstände wurden direkt über eine Protein G Matrix geleitet. Der Proteinnachweis im Durchlauf bzw. Eluat erfolgte über die Messung der optische Dichte bei 280nm. Nach einem Waschschritt mit 150 mM PBS, pH 7,2 bis zum Erreichen des Detektor-Hintergrundwertes wurde mit 0,1M Glycin/HCl, pH 3,3 eluiert. Die Regeneration der Protein G Matrix erfolgte mit 0,1 M Glycin/HCl, pH 2,7.

### Beispiel 7: Herstellung von Konjugaten

### Kopplung von mAK an Peroxidase (POD) zur Verwendung im ELISA

Die Kopplung der mAK an Peroxidase (POD) erfolgte extern. Poly-POD-Konjugate wurden von der Firma MicroCoat (Bernried, Deutschland), HPR (horseraddish peroxidase)-Dextran-Konjugate von der Firma DAKO (Kopenhagen, Dänemark) bezogen.

### Kopplung von mAk an Biotin zur Verwendung im ELISA

Die monoklonalen Antikörper wurden im Anschluß an die Reinigung biotinyliert, um sie im ELISA als Detektionsantikörper einsetzen zu können. Die Kopplung monoklonaler Antikörper an Biotin und POD erfolgte nach bekannten Methoden (Harlow & Lane, 1988).

Die monoklonalen Antikörper wurden bei einer Konzentration von ca. 1-2 mg/ml konjugiert. Vor der Kopplung wurden die Antikörper durch Dialyse in 0,1 M Natrium-Acetat-Puffer, pH 8,3 bzw. 0,1 M Natriumhydrogencarbonat-Puffer, pH 8,3, umgepuffert. Zu je 1 mg Antikörper wurden 50 µg N-Hydroxysuccinimidobiotin (NHS-d-Biotin; Sigma) in DMSO pipettiert und vermischt. Die Mischung wurde 1 Stunde bei Raumtemperatur inkubiert. Danach wurden die biotinylierten Antikörper von ungekoppeltem NHS-d-Biotin durch extensive Dialyse gegen 0,15 M PBS, 0,05 % NaN₃, pH 7,5 befreit.

### Kopplung von mAk an kolloidales Gold zur Verwendung in immunologischen Schnelltests

Für eine Verwendung in immunologischen Schnelltests wurde der monoklonale Antikörper (mAk) an kolloidales Gold konjugiert. Dies erfolgte nach bekannten Standardmethoden (Frens, 1973; Geoghegan und Ackerman, 1977; Slot et al., 1985). Zur Herstellung von kolloidalem Gold wurden 200 ml einer 0,01 % Goldchlorid- (HAuCl₄)-Lösung bis zum Kochen erhitzt und durch Zugabe von 2 ml 1 % Natrium-citrat (Na₃C₆H₅O₇) unter weiterem Kochen reduziert.
Zur Kopplung von mAk an kolloidales Gold wurde eine zur Stabilisierung notwendige Menge des IgG mit der Goldlösung vermischt und 15 min bei Raumtemperatur inkubiert. Die optimale IgG-Konzentration und der geeignete pH-Wert für die Kopplung wurden für jeden mAk individuell bestimmt. Zur Stabilisierung des Gold-IgG-Konjugats wurden Polymere oder Protein, beispielsweise bovines Serumalbumin (BSA) in einer Konzentration von 1 % zum Kopplungsansatz zugegeben. Nicht mit IgG gekoppeltes Goldkolloid und freies IgG wurden anschließend durch Zentrifugieren vom Gold-lgG-Konjugat aus dem Kopplungsansatz entfernt. Zur Lagerung, die bevorzugt bei 4 °C erfolgt, wurde dem Lösungspuffer des Gold-lgG-Konjugats 0,05 % NaN₃ zugegeben.

### Beispiel 8: Charakterisierung der gereinigten monoklonalen Antikörper

### Charakterisierung von Antikörper-Antigen-Wechselwirkungen mittels Oberflächenplasmonresonanz-Spektroskopie (SPR-Spektroskopie)

Mit der SPR-Spektroskopie können die Affinitätskonstanten der monoklonalen Antikörper bestimmt werden. Dadurch lassen sich geeignete Antikörper für die Entwicklung von ELISA und Schnelltest finden.

### Durchführung der Oberflächenplasmonresonanz-Spektroskopie am Pharmacia BIAcore

Alle Schritte wurden auf einer Pharmacia BIAcore Processing Unit CA 186 nach Vorschrift des Herstellers durchgeführt (BlAcore Methods Manual).
Katalase wurde dabei über Aminkopplung auf der Dextranmatrix des BIAcore CM5 Sensorchips immobilisiert. Zur Aktivierung der Dextranmatrix wurden 45 µl einer 1:1-Mischung aus 0,05 M N-Hydroxysuccinimid (NHS) und 0,2 M 1-Ethy-3-(3-dimethylaminopropyl)carbodiimid (EDC)-Lösung bei einer Flußrate von 5 µl/min über den Sensorchip geleitet. Anschließend wurde Katalase (35 µl; 50 µg/ml in 10 mM Natrium-Acetat pH 5,0) an die Dextranmatrix gebunden. Verbleibende NHS-Ester wurden mit 1 M Ethanolamin (35 µl) deaktiviert. Nicht-kovalent an die Dextranmatrix gebundene Katalase wurde durch Regeneration des Sensorchips mit HCI (10 mM; 15 µl) entfernt.

Durch Zugabe der Katalase-spezifischen monoklonalen Antikörper wurden diese mit immobilisierter Katalase zur Reaktion gebracht und die Massenanlagerung am Detektor gemessen. Es wurden Antikörper-Lösungen unterschiedlicher Konzentration im Bereich zwischen 20 und 670 nM eingesetzt. Diese wurden mit einer Flußrate von jeweils 25 µl/min über die auf dem Sensorchip CM5 immobilsierte Katalase injiziert.

### Ergebnisse

Aus dem zeitlichen Verlauf des Resonanzsignals lassen sich die Werte für die Geschwindigkeitskonstanten der Adsorption (kₒₙ) und Desorption (k_{off}) des Antikörpers errechnen (BIAevaluation software 3.0). Es wurden 6 monoklonale Antikörper gegen Katalase bezüglich ihrer Affinitäten getestet:

**Tabelle 1:**

| Ergebnisse der Affinitätsbestimmung der Katalase-mAk | | | |
|---|---|---|---|
| **mAk** | **kₒₙ [M⁻¹ s⁻¹]** | **k_{off} [s⁻¹]** | **K_{D} [M]** |
| HP25.2m/2H10 | 1,44E+05 | 3,90E-05 | 2,71 E-10 |
| HP25.6m/1G4 | 1,41E+05 | 2,52E-05 | 1,79E-10 |
| HP25.6m/1B5 | 5,67E+04 | 3,86E-05 | 6,81E-10 |
| HP25.6m/4E3 | 4,92E+04 | 5,96E-05 | 1,21E-09 |
| HP25.6m/1A5 | 3,91E+04 | 4,77E-05 | 1,22E-09 |
| HP25.6m/1H4 | 7,12E+04 | 4,12E-05 | 5,79E-10 |
| K_{D}= k_{off} : kₒₙ | | | |

### Auswahl von Antikörperpaaren für die Verwendung im ELISA am menschlichen Stuhl

Diejenigen Antikörper, welche die niedrigsten Nachweisgrenzen bei der Messung aus dem Kulturüberstand zeigten, wurden mittels Oberflächen-Plasmonenresonanz Epitop-Überlappungen bestimmt und Affinitätskonstanten gemessen. Die Kombinationen, die sich bei diesen Messungen vielversprechend zeigten (keine Epitop-Überlappung, hohe Geschwindigkeitskonstante für Adsorption, niedrige Geschwindigkeitskonstante der Desorption) wurden auf ihre Antigen-Nachweisgrenze im Sandwich-Stuhl-ELISA getestet.

### Beispiel 9: Screening von mAk Kulturüberständen auf Patientenproben (gemischtes polyklonales / monoklonales System)

Diejenigen monoklonalen Antikörper, die im Fusionsscreening mittels direktem ELISA (Beispiel 4) eine spezifische Antigenerkennung zeigten, wurden als Kulturüberstände im Sandwich-ELISA hinsichtlich ihrer Patienten-Erkennung und Antigen-Nachweisgrenze untersucht.
Als interne Entwicklungsproben standen Stuhlproben zur Verfügung, deren Infektionsstatus (Gruppe 0 und 4) mittels histologischer Untersuchung und / oder ¹³C Urea Atemtest erhoben wurde. Patienten der Gruppe 0 zeigen ein *H. pylori* negatives Ergebnis, Patienten der Gruppe 4 zeigten ein *H. pylori* positives Ergebnis im Referenztest.
Die Beschichtung der ELISA-Platten (Mikrotiterplatte MaxiSorb; Nunc) erfolgte über Nacht bei 5ºC mit 100 µl einer Lösung eines polyklonalen Kaninchen-anti-*H. pylori*-Antikörpers (pAk; ca. 20µg IgG/ml 0,1 M Carbonat-Puffer, pH 9,5). Zur Blockade der noch freien Bindungsstellen wurden 200µl 150mM PBS, pH 7,2 mit 0,2% Fischgelatine (w / v) pro Napf pipettiert und 30min bei Raumtemperatur inkubiert. Danach wurde die ELISA-Platte 2x mit 250µl PBS unter Zusatz von 0,025% Tween 20 (Waschpuffer 1) gewaschen. Humanstuhl wurde im Verhältnis 1:10 (w / v) mit 150mM PBS unter Zugabe von 2% Magermilchpulver und 1mM EDTA suspendiert.
Zur Bestimmung der Antigen-Nachweisgrenze wurde eine *H. pylori* negative Stuhlsuspension mit 50 ng/ml Katalase (siehe Beispiel 3) versetzt und in 1:2 Schritten mit einer *H. pylori* negativen Stuhlsuspension verdünnt. Je 100µl der Stuhlsuspension wurden pro Napf für eine Stunde inkubiert (Doppelbestimmung bei Patientenproben). Die ELISA-Platte wurde ausgeschlagen, mit Waschpuffer 2 (PBS mit 0,2% Tween 20) abgespült und 4x mit Waschpuffer 2 gewaschen. Anschließend wurden 100µl Kulturüberstand von Hybridomen (1:5 in PBS verdünnt) zugegeben und für 60min bei Raumtemperatur inkubiert. Die Detektion der gebundenen Antikörper erfolgte durch Zugabe eines Peroxidase-konjugierten Sekundärantikörpers (Kaninchen-anti-Maus IgG-POD, DAKO). Die Peroxidase setzt im nächsten Schritt das zugegebene farblose POD-Substrat Tetramethylbenzidin (TMB, Sigma) in ein blaues Produkt um. Nach 5-10 Minuten, bevorzugt nach 10 Minuten wurde die Enzym-Reaktion durch Zugabe von 1N Schwefelsäure (100 µl/Napf) gestoppt. Die Stärke der Farbreaktion wurde im ELISA-Reader (MWG Spektral) gemessen. Die Messung erfolgt bei 450 nm gegen die Referenzwellenlänge 620 nm, bevorzugt 630 nm. Vor Zugabe des Detektionsantikörpers bzw. der Substratlösung wurde die ELISA-Platte jeweils 3x bzw. 4x mit Waschpuffer 1 gewaschen.

Als Nachweisgrenze wird ein Extinktionswert, der größer oder gleich dem Zweifachen des Null-Wertes *(H. pylori* negative Stuhlprobe ohne Antigen-Zumischung) ist, festgelegt.
Der monoklonale Antikörper HP25.2m/2H10 zeigte im Sandwich-ELISA unter der Verwendung eines polyklonalen Fänger-Antikörpers, der gegen *H. pylori* Lysat gerichtet ist, eine Sensitivität von 68% (von 25 positiven Proben wurden 17 richtig delektiert) und eine Spezifität von 82% (von 17 HP-negativen Proben wurden 3 Proben falsch positiv detektiert). Das Patienten-Erkennungsverhalten weiterer monoklonaler Antikörper (Kulturüberstände) ist aus Tabelle 3 zu entnehmen.

**Tabelle 2:**

| **HP25.2m/2H10: Sensitivität und Spezifität im Sandwich-ELISA (Fang-Antikörper pAK gegen *H. pylori*)** | | | |
|---|---|---|---|
| **Stuhlprobe** | **Infektions-Status des Patienten** | **Fänger-AK: pAK gegen HP Detektions-AK: HP25.2m/2H10 (Kulturüberstand)** **OD 450-630** | **Auswertung cut off: 0,1: OD₄₅₀₋₆₃₀= 0,1** |
| CX0010 | POSITIV | 0,25 | positiv |
| CX1014 | POSITIV | 0,75 | positiv |
| CX1029 | POSITIV | 0,18 | positiv |
| CX1038 | POSITIV | 0,09 | negativ |
| CX1052 | POSITIV | 0,11 | positiv |
| CX2008 | POSITIV | 0,63 | positiv |
| CX2009 | POSITIV | 0,32 | positiv |
| CX2016 | POSITIV | 0,07 | negativ |
| CX2019 | POSITIV | 0,59 | positiv |
| CX2029 | POSITIV | 0,52 | positiv |
| CX0213 | POSITIV | 0,04 | negativ |
| CX294-1 | POSITIV | 0,14 | positiv |
| CX3098 | POSITIV | 0,13 | positiv |
| CX3146 | POSITIV | 0,05 | negativ |
| CX3148 | POSITIV | 0,08 | negativ |
| CX3234 | POSITIV | 0,18 | positiv |
| CX4003 | POSITIV | 0,17 | positiv |
| CX4006 | POSITIV | 0,25 | positiv |
| CXT001 | POSITIV | 0,23 | positiv |
| CXT002 | POSITIV | 0,53 | positiv |
| CXT003 | POSITIV | 0,12 | positiv |
| CXT004 | POSITIV | 0,03 | negativ |
| CXT005 | POSITIV | 0,03 | negativ |
| CXT006 | POSITIV | 0,31 | positiv |
| CXT007 | POSITIV | 0,08 | negativ |
| CX1008 | NEGATIV | 0,29 | positiv |
| CX1031 | NEGATIV | 0,08 | negativ |
| CX1049 | NEGATIV | 0,7 | positiv |
| CX1051 | NEGATIV | 0,09 | negativ |
| CX0142 | NEGATIV | 0,03 | negativ |
| CX0185 | NEGATIV | 0,03 | negativ |
| CX0189 | NEGATIV | 0,08 | negativ |
| CX0193 | NEGATIV | 0,03 | negativ |
| CX2010 | NEGATIV | 0,08 | negativ |
| CX2018 | NEGATIV | 0,09 | negativ |
| CX0220 | NEGATIV | 0,03 | negativ |
| CX0231 | NEGATIV | 0,03 | negativ |
| CX0258 | NEGATIV | 0,02 | negativ |
| CX3008 | NEGATIV | 0,09 | positiv |
| CX3011 | NEGATIV | 0,08 | negativ |
| CX3033 | NEGATIV | 0,07 | negativ |
| CX3035 | NEGATIV | 0,09 | negativ |
| Abkürzungen: pAK: polyklonaler Antikörper; HP: *H. pylori* | | | |

**Tabelle 3:**

| Charakterisierung der monoklonalen Antikörper gegen Katalase | | | | | |
|---|---|---|---|---|---|
| **Fusion/Klon** | **Isotyp** | **WB (Ag)** | **NWG (ng/ml)** | **Stuhlproben, die korrekt erkannt wurden** | |
| | | | | pos. Proben | neg. Proben |
| HP25.2m/2H1 0 | IgG2a, κ | + | 1,5 | 17 von 25 | 14 von 17 |
| HP25.6m/1G4 | IgG1, κ | + | 1,5 | 4 von 5 | 2 von 2 |
| HP25.6m/1B5 | IgG1, κ | + | 3-6 | 3 von 5 | 2 von 2 |
| HP25.6m/1H4 | IgG1, κ | + | 3-6 | 2 von 5 | 2 von 2 |
| HP25.6m/4E3 | IgG1, κ | + | 6 | 2 von 5 | 2 von 2 |
| HP25.6m/1A5 | IgG1, κ | + | 6 | 2 von 5 | 2 von 2 |
| HP25.6m/5E4 | IgG1, κ | - | 1,5 | 1 von 5 | 2 von 2 |
| HP25.6m/4A1 2 | IgG1, κ | - | 1,5 | 1 von 5 | 2 von 2 |
| HP25.6m/5F4 | IgG1, κ | - | 1,5 | 1 von 5 | 2 von 2 |
| Abkürzungen: Ag Antigen; WB Westernblot; NWG Nachweisgrenze | | | | | |

### Ergebnisse

Tabelle 3 faßt die Ergebnisse der Isotyp-Bestimmung, der Westernblot-Analysen, der Nachweisgrenzen-Bestimmung und der Patienten-Erkennung für die monoklonalen Antikörper (mAK) gegen Katalase zusammen. Aus den Daten wird ersichtlich, daß eine gute Erkennung der nativen Katalase mittels mAK nicht mit einer guten Patienten-Erkennung korreliert.
Im gemischt polyklonalen / monoklonalen Sandwich-ELISA-System zeigte der mAK HP25.2m/2H10 eine Sensitivität von 68% und eine Spezifität von 82%. Eine Verbesserung von Sensitivität und Spezifität ist durch den Einsatz von gereinigten mAk (statt Kulturüberstand) in einem rein monoklonalen ELISA-System zu erwarten. Dabei können entweder ein monoklonaler Antikörper, der gegen das gleiche Epitop des Antigens gerichtet ist (siehe Beispiel 10), oder zwei verschiedene monoklonale Antikörper, die gegen verschiedene Epitope des gleichen Antigens gerichtet sind (siehe Beispiel 12), als Fänger- und Detektor-Antikörper eingesetzt werden

### Beispiel 10: Detektion von H. pylori im menschlichen Stuhl mittels ELISA (rein monoklonales System)

Für den Test standen Stuhlproben von Patienten aus zehn verschiedenen Kliniken oder gasteroenterologischen Praxen zur Verfügung, deren *H. pylori*-Status mittels ¹³C Urea Atemtest und / oder histologischer Untersuchungen von Magenbiopsien erhoben wurde. Die zu untersuchenden Stuhl-Proben wurden codiert, so daß dem Laborpersonal der Infektionsstatus nicht bekannt war.

### H. pylori-Stuhl-Sandwich-ELISA (Dreischritt-ELISA)

Die Beschichtung der ELISA-Platten (MaxiSorb; Nunc) erfolgte für 1h bei 37°C mit 100 µl einer mAK-Lösung (2.0 µg HP25.2m/2H10/ml 0.1M Carbonatpuffer, pH 9,5). Zur Blockade der noch freien Bindungsstellen wurden 200µl 150mM PBS mit 0,2% Fischgelatine (w / v) pro Napf pipettiert und 30min bei Raumtemperatur inkubiert. Anschießend folgte ein zweimaliges Waschen mit 250µl Waschpuffer 1 (PBS mit 0,025% Tween). Humanstuhl wurde im Verhältnis 1:10 (w / v) mit 150mM PBS unter Zusatz von 2% Magermilchpulver und 1mM EDTA suspendiert. Zur Bestimmung der Antigen-Nachweisgrenze wurde gereinigte *H. pylori*-Katalase in bekannten Konzentrationen der Stuhlsuspension eines *H. pylori* negativen Patienten zugegeben. Die Stuhlprobensuspensionen wurden 5min bei 7000g abzentrifugiert. Je 100µl des Überstandes wurden pro Napf für 1h inkubiert. Die Platte wurde ausgeschlagen, abgespült und 4x mit Waschpuffer 2 (250µl PBS unter Zusatz von 0,2% Tween) gewaschen. Anschließend wurden 100µl einer Lösung Biotin-gekoppelten mAK (1 µg/ml HP25.2m/2H10-Bio in PBS; 0,1% BSA) zugegeben und für 60min bei Raumtemperatur inkubiert. Die Detektion der gebundenen Antigene erfolgt durch Zugabe eines Konjugats von Streptavidin mit POD (Dianova). Die POD setzt dann im nächsten Schritt das farblose Substrat TMB (Sigma) in ein blaues Produkt um. Nach 5 bis 10 Minuten, bevorzugt 10 Minuten wurde die Enzym-Reaktion durch Zugabe von 1N Schwefelsäure (100 µl/Napf) gestoppt. Die Stärke der Farbreaktion wurde im ELISA-Reader (MWG Spektral) gemessen. Die Messung erfolgt bei 455 nm gegen die Referenzwellenlänge 620nm, oder 630 nm.

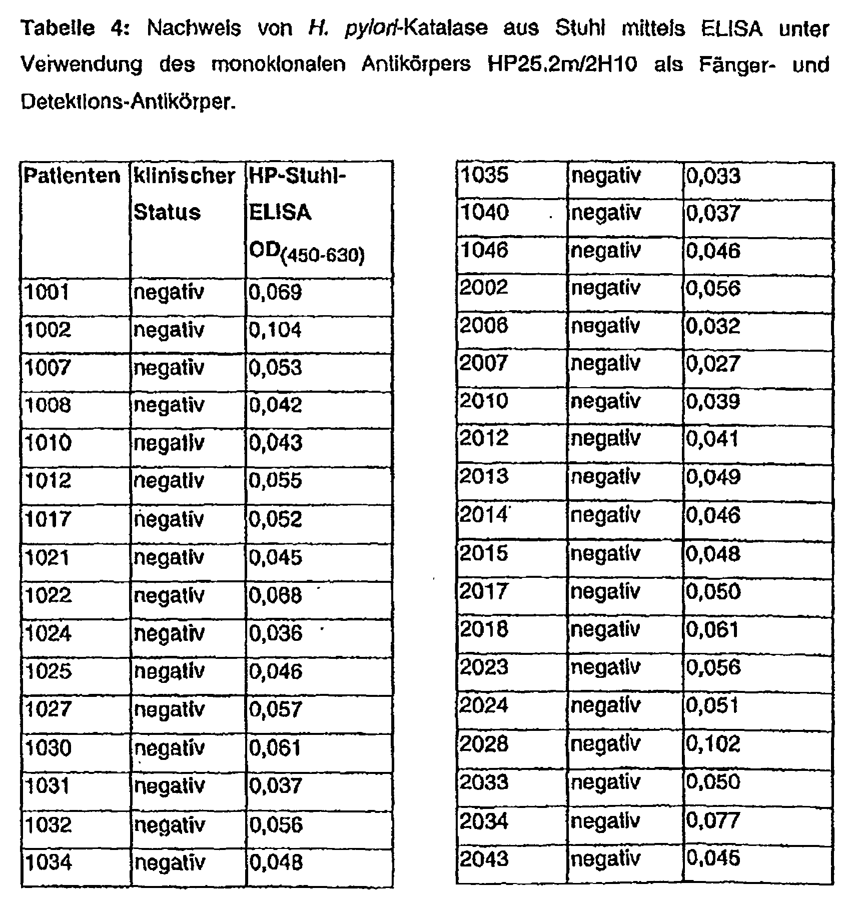

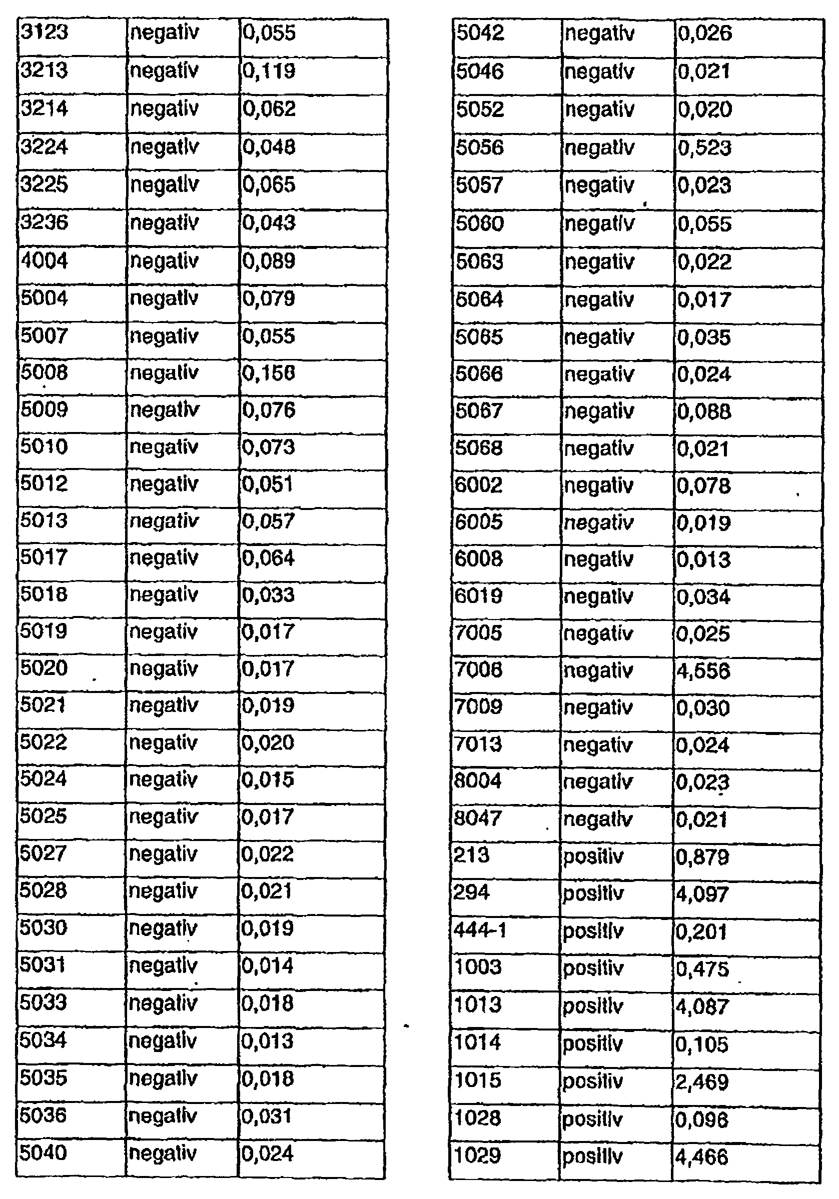

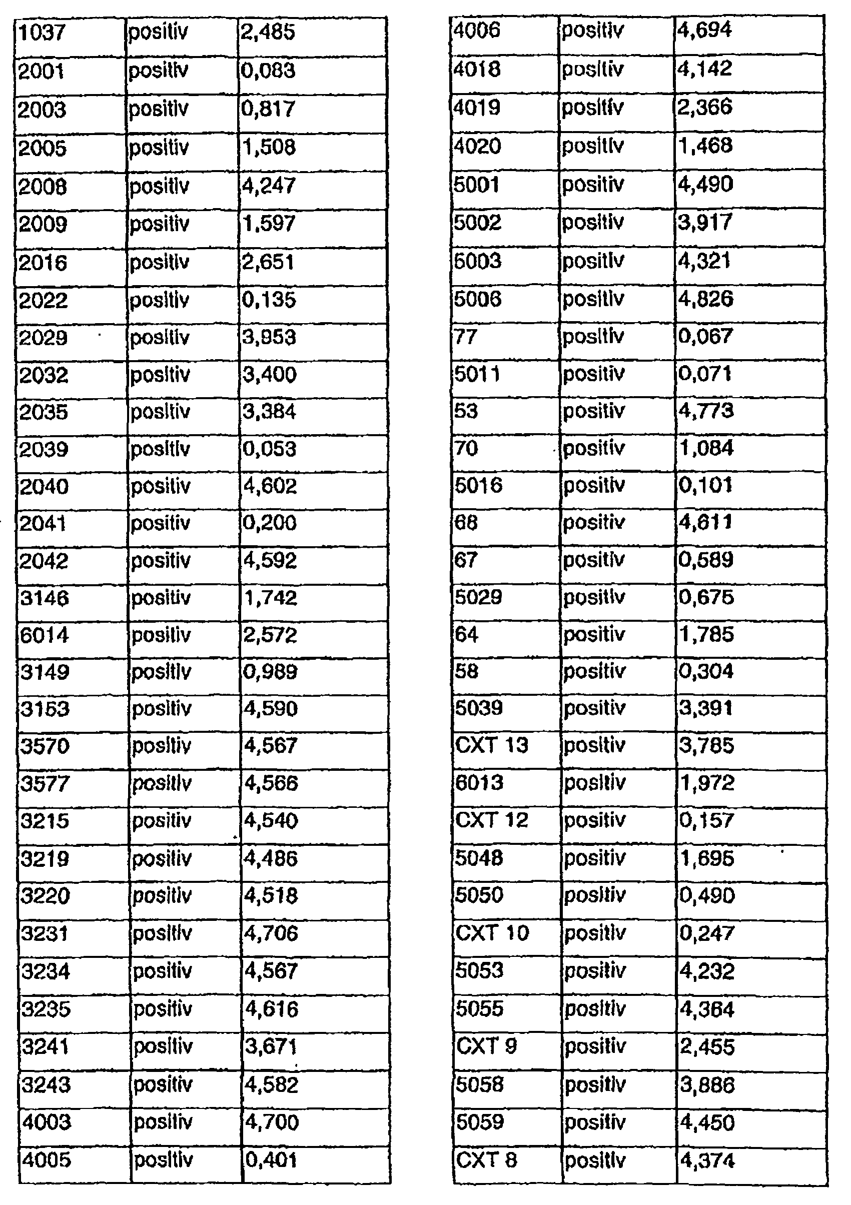

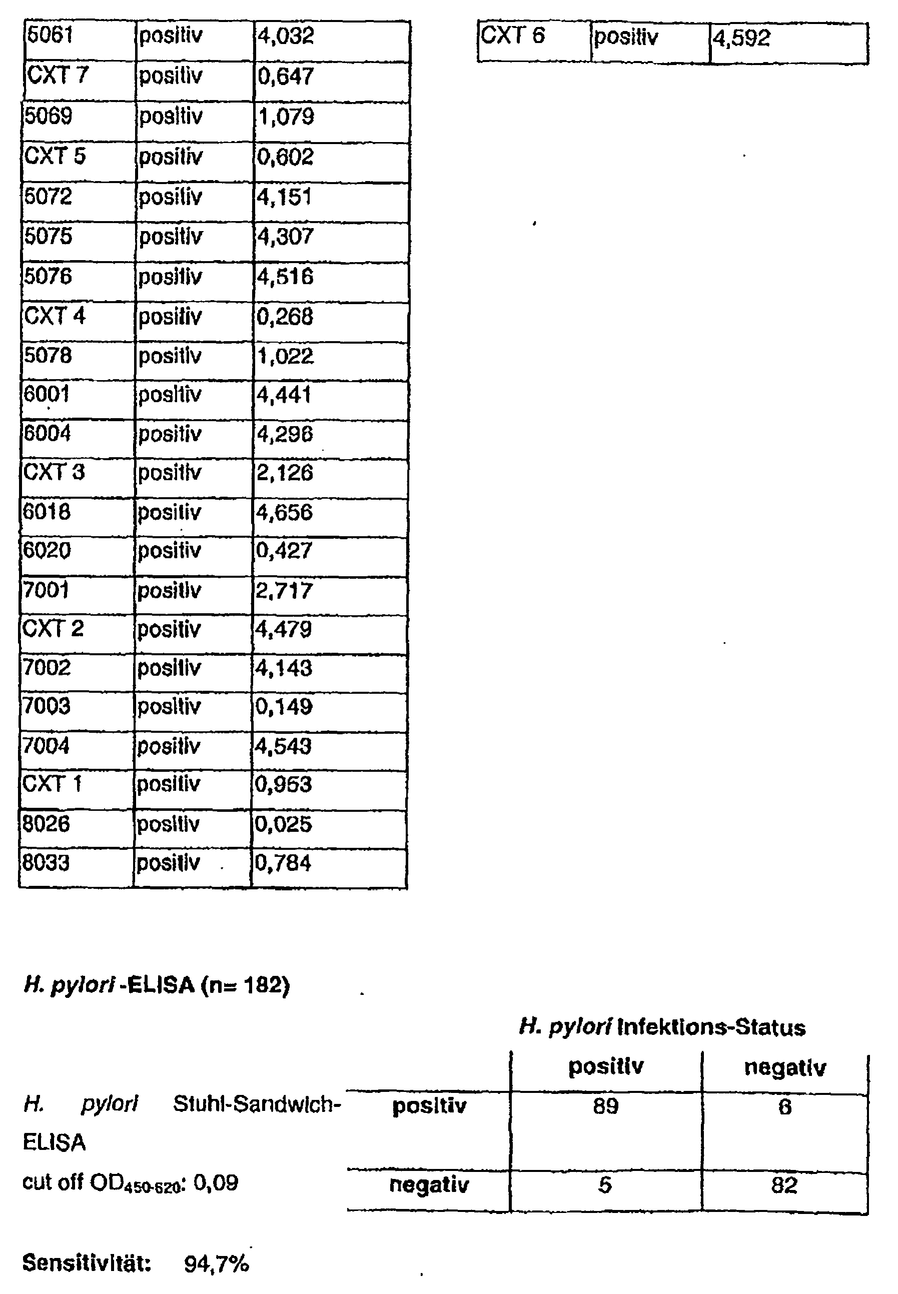

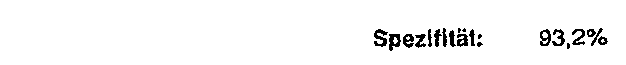

### Ergebnis:

Tabelle 4 zeigt die Ergebnisse der Untersuchung von *H. pylori* negativen und *H*. *pylori* positiven Stuhlproben mittels eines Stuhl Sandwich ELISA. Dabei werden monoklonale Antikörper, bevorzugt der monoklonale Antikörper HP25.2m/2H10 sowohl als Fänger- als auch als Detektions-Antikörper (POD-markiert) zur Detektion des *H. pylori*-Antigens Katalase aus der Stuhlprobe eingesetzt. Bei der Katalase handelt es sich um ein äußerst stabiles Antigen, welches den Verdauungstrakt weitgehend unverändert passiert und somit in der Stuhlprobe nachgewiesen werden kann. Die Untersuchung von 182 Stuhiproben im rein monoklonalen ELISA-System, das auf nur einem Katalase-spezifischen mAk aufgebaut ist, weist eine Sensitivität von 94,7% und eine Spezifität von 93,2% auf. Diese Sensitivität und Spezifität führt zu so hohen positiven und negativen prädiktiven Werten, daß eine Infektion mit H. pylori mit ausreichender Sicherheit schon alleine durch eine einfache, preisgünstige und nicht invasive Stuhluntersuchung festgestellt werden kann, um über eine Eradikationsbehandlung zu entscheiden. Eine Steigerung von Sensitivität und Spezifität kann möglicherweise durch eine Kombination von verschiedenen mAks, die gegen unterschiedliche Epitope der Katalase gerichtet sind oder durch eine Kombination zweier Nachweis-Systeme für verschiedene Antigene (z.B. Katalase/Urease) erzielt werden.
Mit der Entwicklung eines Einschritt-ELISA-Tests wurde gegenüber dem Dreischritt-ELISA-Test, der nachfolgend (Beispiel 11 und 12) beschrieben wird, eine erhöhte Anwenderfreundlichkeit erreicht.

### Beispiel 11: Ermittlung von geeigneten Antikörperpaaren im Dreischritt-ELISA

Die Testdurchführung erfolgte gemäß Beispiel 10.
Zur Ermittlung von geeigneten Antikörperpaaren standen die monoklonalen Antikörper gegen Katalase (siehe Tabelle 3) gereinigt und z.T. biotinyliert (siehe Beispiel 7) zur Verfügung. Die Antikörper wurden zur Ermittlung der optimalen Einsatzkonzentration des Fang- und Detektor-Antikörpers zunächst gegeneinander austitriert. Dann wurden mit den so optimierten ELISA-Systemen Patientenstuhlproben getestet, sowie die Nachweisgrenzen von Katalase in humanem *H. pylori* negativem Stuhl (Nullstuhl) bestimmt (Tab. 5).

Geeignete mAK-Kombinationen bezüglich der Patienten-Erkennung und Antigen-Nachweisgrenze sind in Tabelle 5 aufgeführt.

**Tabelle 5:**

| Ergebnisse der Paarfindung der monoklonalen Antikörper gegen Katalase (Dreischritt-ELISA) | | | | | |
|---|---|---|---|---|---|
| | Fänger-Antikörper | | | | |
| biotinylierter Detektor-Antikörper | 25.2m/2H10 | 25.6m/ 1B5 | 25.6m/ 1G4 | 25.6m/ 1A5 | 25.6m/ 1H4 |
| 25.2m/2H10 | N: 0,03 | 0,1 | 0,03 | 0,1 | 0,03 |
| | G4: 7-8 | 7 | 8 | 7 | 8 |
| | G0: 2 | 2 | 2 | 2 | 2 |
| 25.6m/1B5 | N: 0,1 | 0,1 | 0,1 | 0,03 | 0,3 |
| | G4: 8 | 7 | 5 | 7 | 8 |
| | G0: 2 | 1 | 2 | 2 | 2 |
| 25.6m/1G4 | N: 0,3 | 0,1 | 0,1 | 0,01 | 0,1 |
| | G4: 6-8 | 7 | 8 | 8 | 8 |
| | G0: 1-2 | 2 | 4 | 2 | 2 |
| 25.6m/1A5 | N: 0,3 | 0,1 | 0,3 | 0,1 | 0,3 |
| | G4: 6-7 | 7 | 5 | | 7 8 |
| | G0: 2 | 2 | 2 | 2 | 2 |
| 25.6m/1H4 | N: 0,1 | 0,3 | 0,1 | 0,3 | 0,1 |
| | G4: 8 | | | | 4-7 7 8 |
| | G0: 3 | | | | 2 2 2 |
| Patientenerkennung (Detektion von 8 kritisch positiven G4- und 4 klinisch negativen G0-Proben) N = Nachweisgrenzen [ng/ml] der Katalase in Nullstuhl kritisch positiv = Proben die sich im Nachweis als besonders problematisch erwiesen | | | | | |

### Ermittlung von geeigneten Antikörperpaaren im Einschritt-ELISA

Zur Ermittlung von geeigneten Antikörperpaaren standen monoklonale Antikörper gegen Katalase (siehe Tabelle 3) in gereinigtem Zustand und z.T. Peroxidasemarkiert (siehe Beispiel 7) zur Verfügung.
Die verschiedenen Kombinationen von Fang- und Detektions-Antikörpern (siehe Tabelle 6) wurden im Einschritt-ELISA an 27 *H. pylori*-positiven und 17 *H. pylori*-negativen Patientenproben getestet.

### Einschritt-Sandwich-ELISA:

Die Beschichtung der ELISA-Platte (MaxiSorb Lockwell; Nunc) erfolgte über Nacht bei 2-8 °C mit 100 µl einer mAK-Lösung (2.0 µg Fang-Antikörper / ml Carbonatpuffer, 0,1 M, pH 9,5). Die so beschichteten ELISA-Platten wurden 2x mit PBS gewaschen und zum Blockieren der freien Bindungsstellen mit 200 µl Blockierungspuffer (0,3% BSA; 20% Sorbitol in PBS) je Vertiefung versetzt und bei 2-8 °C über Nacht inkubiert. Diese ELISA-Platten wurden abgesaugt, über Nacht bei 28°C im Umluft-Trockenschrank getrocknet und anschließend mit Trockenmittel-Beutel bei 2-8 °C gelagert.
Patientenstuhl wurde 1:5 (0,1g Stuhlprobe + 500µl Probenpuffer) in Probenpuffer (150mM PBS + 0,5% Tierserum + 1mM EDTA + 0,1% Detergens) ca. 30 sec suspendiert (Vortex) und anschließend 5 min bei 3000 g zentrifugiert. Pro Napf wurden 50 µl des Überstands auf die Platte aufgetragen.
Anschließend wurden 50 µl des in Probenpuffer verdünnten POD-markierten Antikörpers (0,5 nM AK-Dextran-POD bzw. 0,2 µg/ml HP25.2m/2H10-POD-P) direkt in die Stuhlsuspension gegeben. Die Platten wurden 1 Stunde bei Raumtemperatur auf dem Schüttler inkubiert.
Nach fünfmaligen Waschen mit Waschpuffer (75 mM PBS, 0,25% Tween) wurde das Peroxidase-Substrat TMB (Tetramethylbenzidin) Einkomponentensubstrat (Neogen) zugegeben (100 µl/Napf). Nach 10 Minuten wurde die Enzymreaktion durch Zugabe von 1N Salzsäure (100 µl/Napf) gestoppt. Die anschließende Messung der Farbintensität erfolgte bei 450 nm gegen die Referenzwellenlänge 630 nm.

**Tabelle 6:**

| Ergebnisse der Paarfindung der monoklonalen Antikörper gegen Katalase (Einschritt-ELISA) | | | | | | | | | |
|---|---|---|---|---|---|---|---|---|---|
| | Fänger-Antikörper | | | | | | | | |
| POD markierter Detektor- Antikörper | 25.2m/ 2H10 | 25.6m/ 1B5 | 25.6m/1A5 | 25.6m/ 4A12 | 25.6m/ 1G4 | 25.6m/1H4 | 25.6m/ 3D6 | 25.6m/ 2E12 | 25.6m/ 5E4 |
| 25.2m/2H10 | - | 24 | 24 | 22 | 23 | 24 | 19 | 24 | 22 |
| | | 14 | 11 | 13 | 14 | 12 | 14 | 12 | 15 |
| 25.6m/1B5 | G4: 23 | - | 23 | 20 | 23 | 23 | 18 | 23 | 22 |
| | G0: 12 | | 13 | 14 | 12 | 12 | 12 | 10 | 12 |
| 25.6m/1H4 | G4: 21 | 24 | 24 | - | 24 | - | 20 | 25 | 24 |
| | G0: 9 | 13 | 14 | | 11 | | 15 | 12 | 20 |
| 25.6m/4A12 | G4: 20 | 20 | 20 | - | 20 | 25 | 19 | 20 | 20 |
| | G0: 13 | 12 | 17 | | 13 | 3 | 13 | 13 | 15 |
| 25.6m/3D6 | G4: 17 | 24 | 24 | 21 | 23 | 23 | - | 22 | 22 |
| | G0: 15 | 9 | 12 | 13 | 8 | 13 | | 9 | 14 |
| Patientenerkennung (Detektion von 27 kritisch positiven G4- und 17 klinisch negativen G0-Proben); Cut off OD ₄₅₀₋₆₃₀ₙₘ: 0,15 | | | | | | | | | |

Die Antikörperkombination HP25.6m/1B5 (Fang-Antikörper) und HP25.2m/2H10-POD (Detektions-Antikörper) erwies sich aufgrund der guten Patientenerkennung (korrekte Detektion von 24 aus 27 *H. pylori* positiven und 14 aus 17 *H. pylori* negativen Proben) sowie der hohen Signalstärke zum Nachweis von *H. pylori* Antigenen (Katalase) in Humanstuhl am vorteilhaftesten.

### Beispiel 12: Detektion von H. pylori im menschlichen Stuhl mittels Einschritt - ELISA

Für den Test standen Stuhlproben von Patienten aus zehn verschiedenen Kliniken oder gasteroenterologischen Praxen zur Verfügung, deren *H. pylori* negativ oder *H*. *pylori* positiv Status) mittels ¹³C Urea Atemtest und / oder histologischer Untersuchungen von Magenbiopsien erhoben wurde.

### H. pylori-Stuhl-Sandwich-ELISA (Einschritt-Test)

Die Beschichtung der ELISA-Platte (MaxiSorb Lockwell; Nunc) erfolgte über Nacht bei 2-8 °C mit 100 µl einer mAK-Lösung (2.0 µg HP25.6m/1B5 / ml Carbonatpuffer, 0,1 M, pH 9,5). Die so beschichteten ELISA-Platten wurden 2x mit PBS gewaschen. Zum Blockieren der freien Bindungsstellen wurden 200 µl Blockierungspuffer (0,3% BSA; 20% Sorbitol in PBS) je Napf zugegeben und bei 2-8 °C über Nacht inkubiert. Diese Platten wurden abgesaugt, über Nacht bei 28°C im Umluft-Trockenschrank getrocknet und anschließend mit Trockenmittel-Beutel bei 2-8 °C gelagert.
Patientenstuhl wurde 1:5 (0,1g Stuhlprobe + 500µl Probenpuffer) in Probenpuffer (150mM PBS + 0,5% Tierserum + 1mM EDTA + 0,1% Detergens) ca. 30 sec suspendiert (Vortex) und anschließend 5 min bei 3000 g zentrifugiert. Pro Napf wurden 50 µl des Überstands (Doppel- bis Dreifachbestimmung) auf die Platte aufgetragen. Anschließend wurden 50 µl des in Probenpuffer-verdünnten POD-markierten Antikörpers HP25.2m/2H10-Dextran-POD direkt in die Stuhlsuspension gegeben. Die Platten wurden 1 Stunde bei Raumtemperatur auf dem Schüttler inkubiert.
Nach fünfmaligen Waschen mit Waschpuffer (75 mM PBS, 0,25% Tween) wurde Peroxidase-Substrat TMB (Tetramethylbenzidin) Einkomponentensubstrat (Neogen) zugegeben (100 µl/Napf). Nach 10 Minuten wurde die Enzymreaktion durch Zugabe von 1N Salzsäure (100 µl/Napf) gestoppt. Die anschließende Messung der Farbintensität erfolgte bei 450 nm gegen die ReferenzVertiefungenlänge 630 nm.

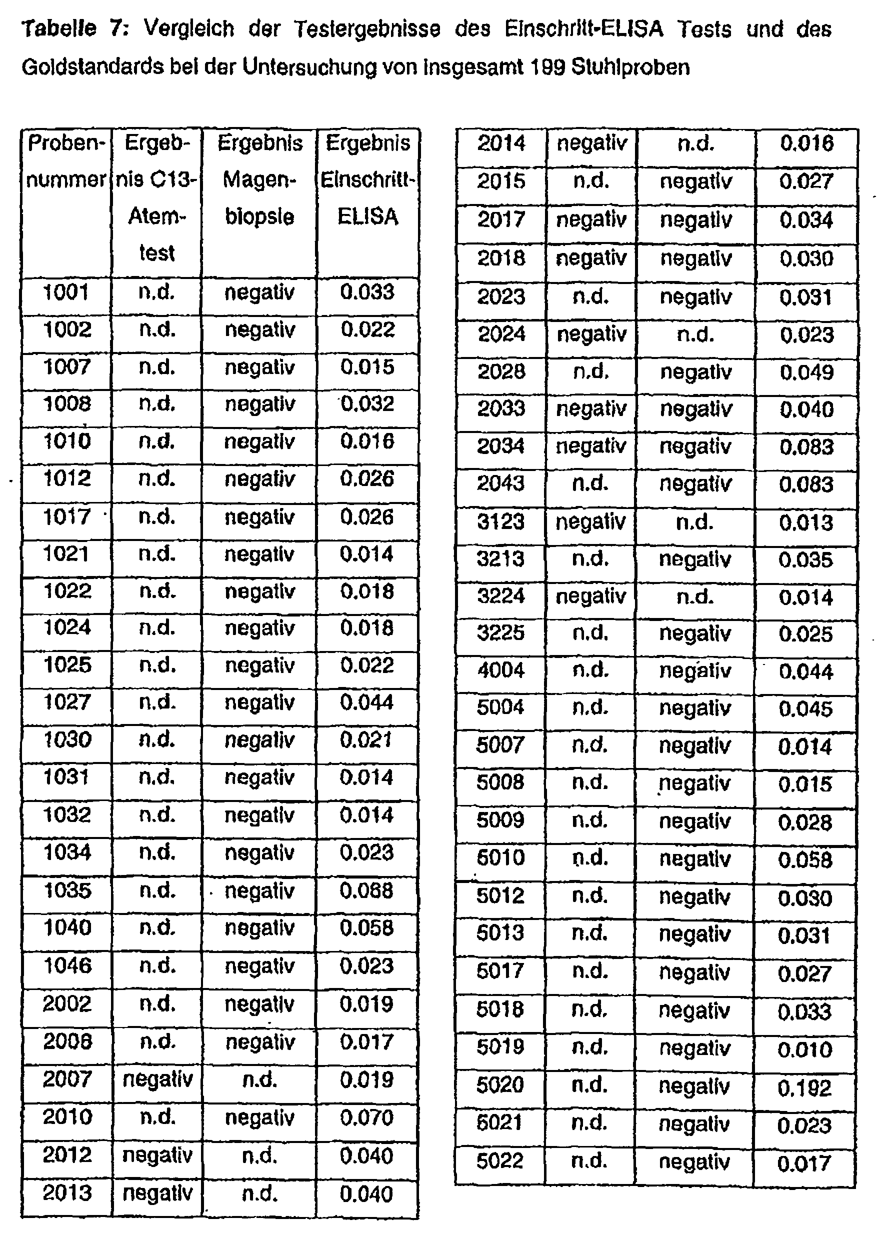

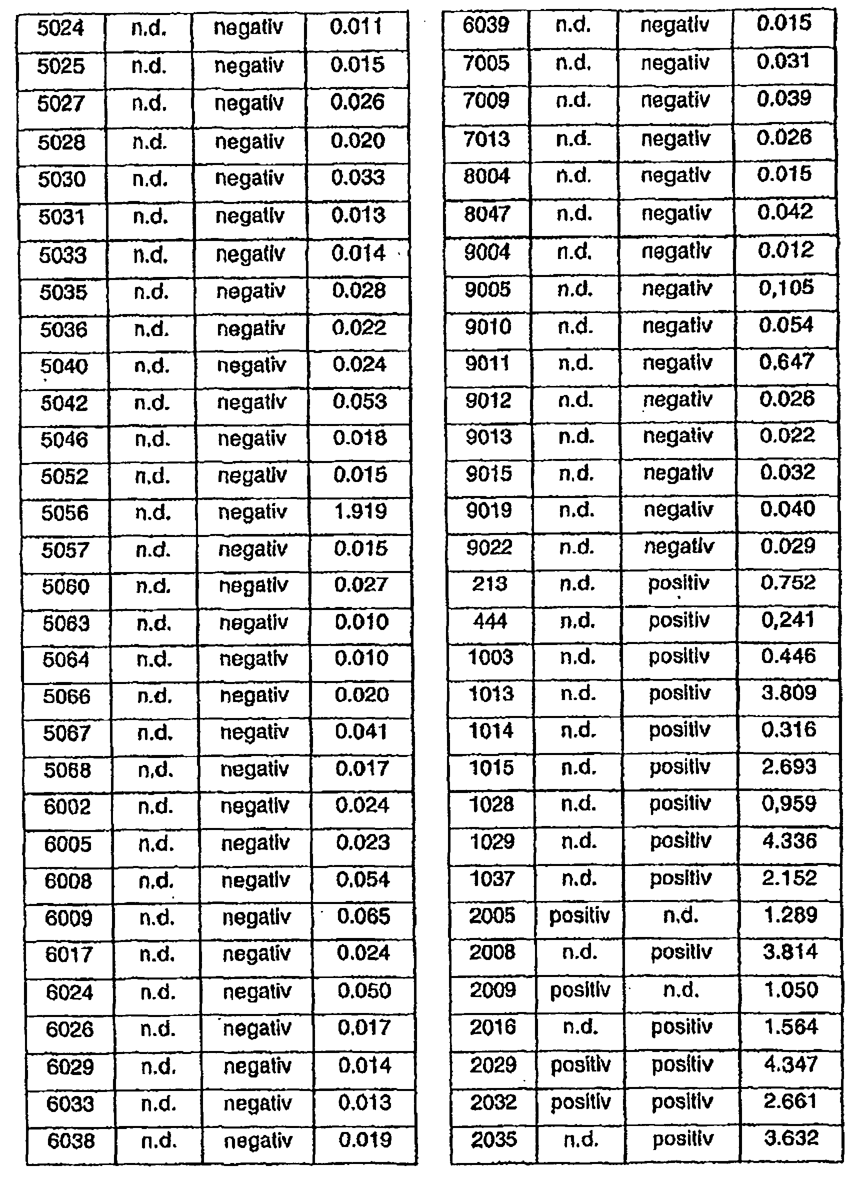

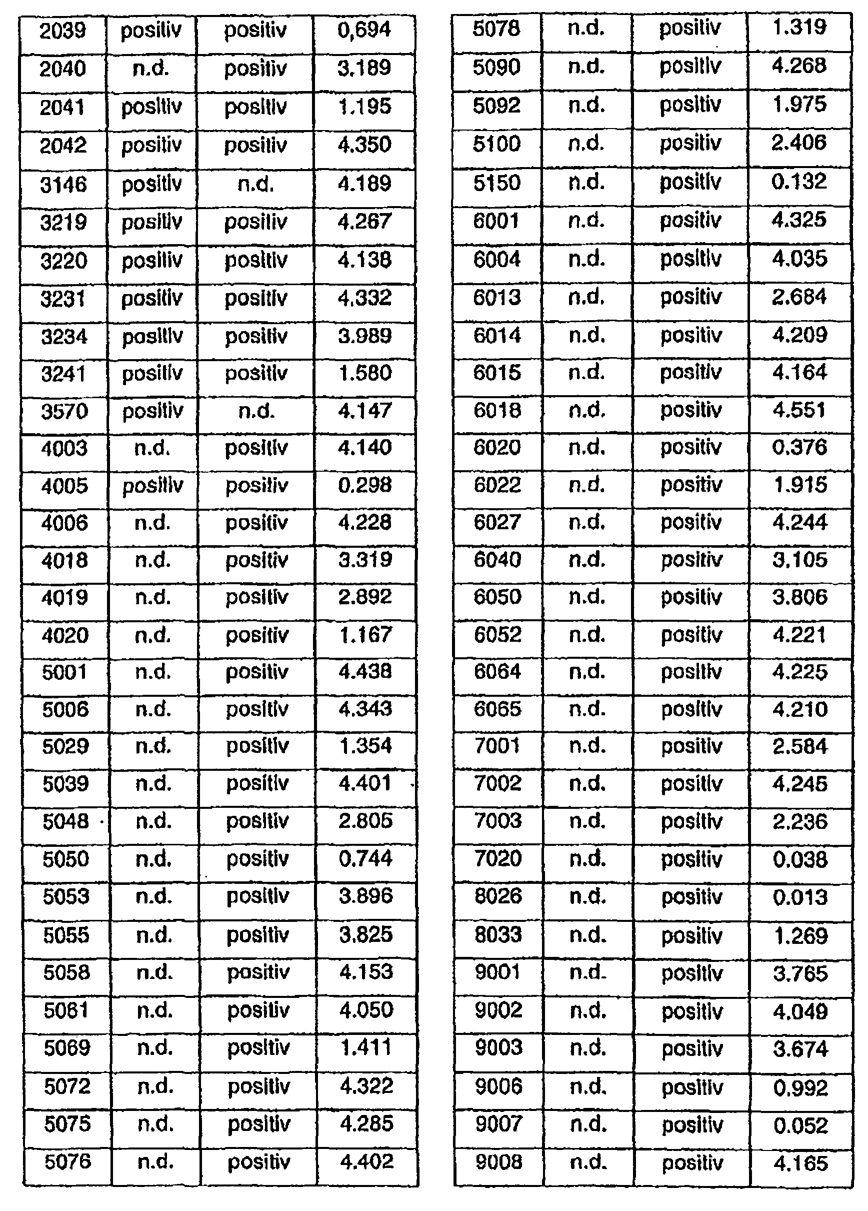

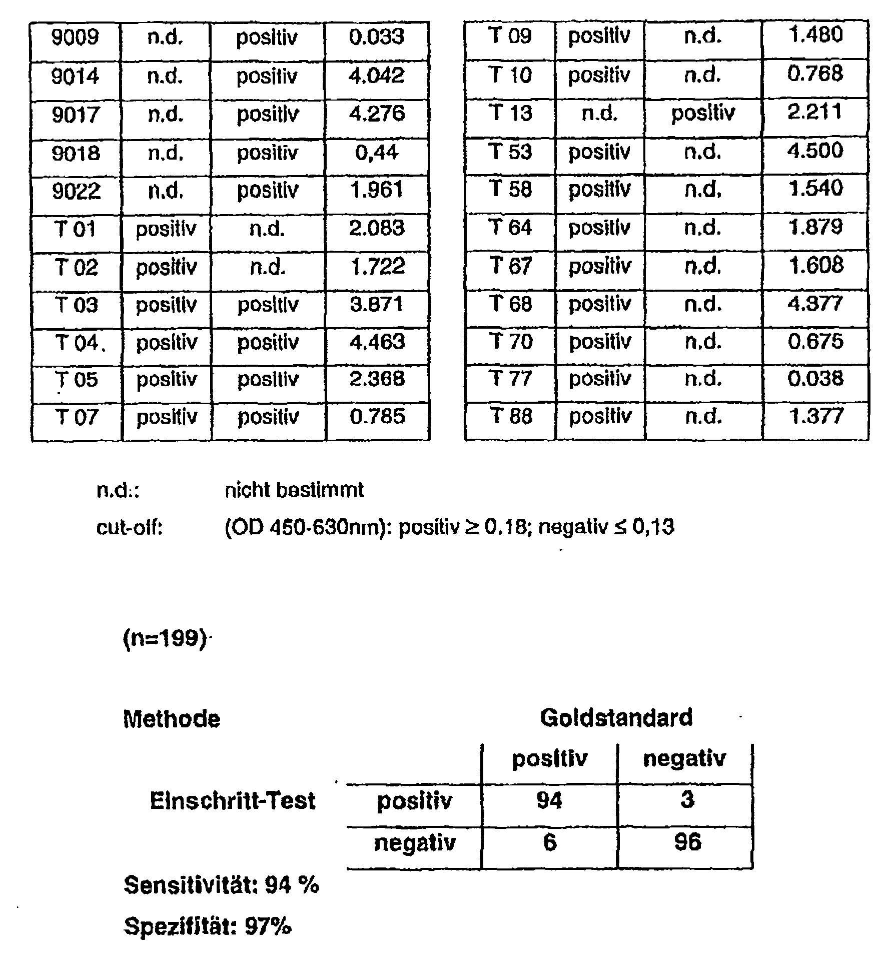

### Ergebnis:

Tabelle 7 zeigt die Ergebnisse der Untersuchung von *H. pylori* negativen und *H. pylori* positiven Stuhlproben mittels eines Einschritt-Stuhl Sandwich ELISA. Dabei wurden die monoklonalen Antikörper (HP25.6m/1B5; HP25.2m/2H10) für die Detektion des *H. pylori*-Antigens Katalase aus der Stuhlprobe eingesetzt. Die Untersuchung von 199 Stuhlproben im rein monoklonalen ELISA-System, das auf oben benannten Katalase-spezifischen mAK aufgebaut ist, weist eine Sensitivität von 94 % und eine Spezifität von 97% auf.

### Beispiel 13: Detektion von H. pylori im menschlichen Stuhl mittels optimiertem Einschritt-ELISA

Für den Test standen 357 Stuhlproben von Patienten aus zehn verschiedenen Kliniken oder gasteroenterologischen Praxen zur Verfügung, deren *H. pylori* Status mittels histologischer Untersuchung von Magenbiopsien erhoben wurde. Die Testdurchführung erfolgte verblindet (die Testproben wurden so codiert, daß dem Laborpersonal der Infektionsstatus der Proben nicht bekannt war) in einem externen GLP-Labor.

### Optimierter Einschritt-Sandwich-ELISA:

Die Beschichtung der ELISA-Platte (MaxiSorb Lockwell; Nunc) erfolgte über Nacht bei 2-8 °C mit 100 µl einer mAK-Lösung (2.0 µg HP25.6m/1B5 / ml Carbonatpuffer, 0,1 M, pH 9,5). Die so beschichteten ELISA-Platten wurden 2x mit PBS gewaschen, zum Blockieren der freien Bindungsstellen mit 200 µl Blockierungspuffer (0,3% BSA; 5% Sorbitol in PBS) je Napf versetzt und bei 2-8 °C über Nacht inkubiert. Diese Platten wurden abgesaugt, über Nacht bei 28°C im Umluft-Trockenschrank getrocknet und anschließend mit Trockenmittel-Beutel bei 2-8 °C gelagert.
Patientenstuhl wurde 1:5 (0,1g Stuhlprobe + 500µl Probenpuffer) in Probenpuffer (150mM PBS + 0,5% Tierserum + 1mM EDTA + 0,1 % Detergens) ca. 30 sec suspendiert (Vortex) und anschließend 5 min bei 3000 g zentrifugiert. Pro Napf wurden 50 µl des Überstands auf die Platte aufgetragen. Anschließend wurden 50 µl des in Probenpuffer verdünnten Peroxidase-markierten Antikörpers (200 femtomol / ml HP25.2m/2H10-Dextran-POD-markiert) direkt zur Stuhlsuspension zugegeben. Die Platten wurden 1 Stunde bei Raumtemperatur auf dem Schüttler inkubiert. Nach fünfmaligen Waschen mit Waschpuffer (75 mM PBS, 0,25% Tween) wurde das Peroxidase-Substrat TMB (Tetramethylbenzidin) Einkomponentensubstrat (Seramun) zugegeben (100 µl/Napf). Nach 10 Minuten wurde die Enzymreaktion durch Zugabe von 1M Schwefelsäure (100 µl/Napf) gestoppt. Die anschließende Messung der Farbintensität erfolgte bei 450 nm gegen die Referenzwellenlänge 630 nm.

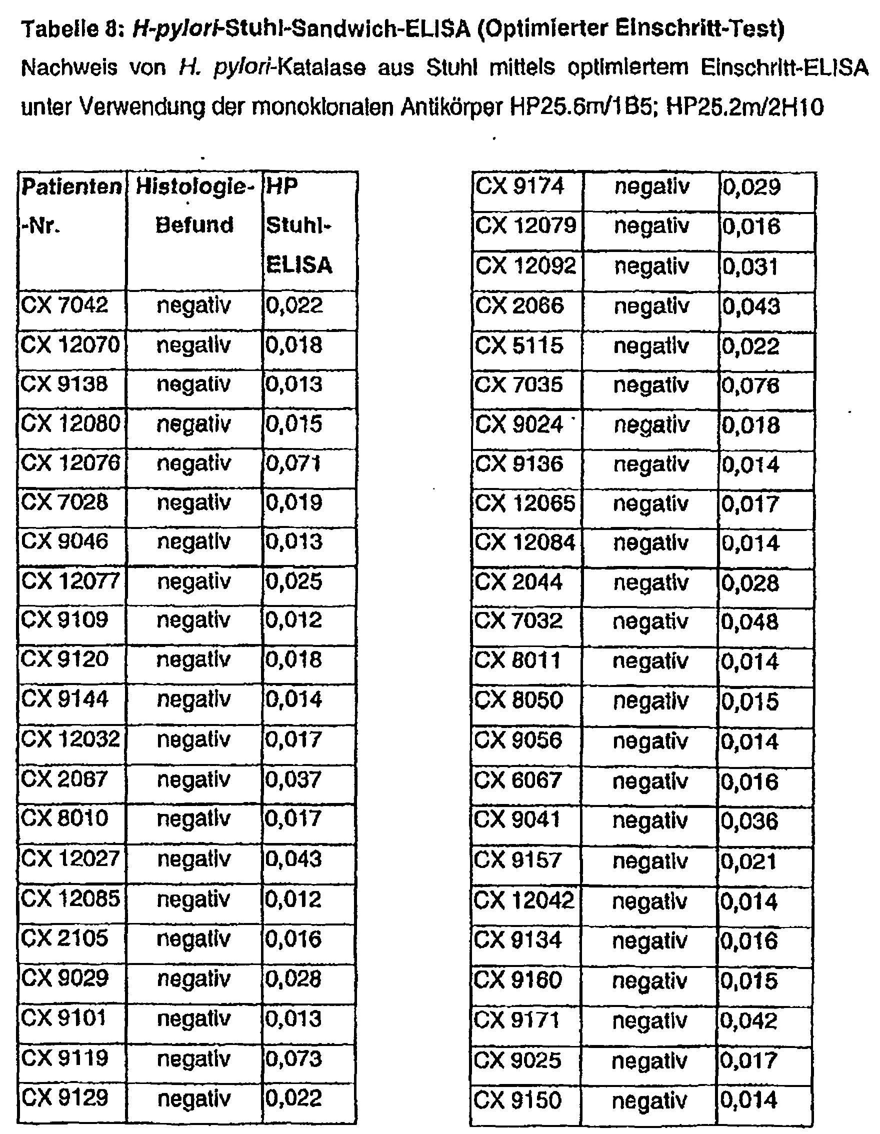

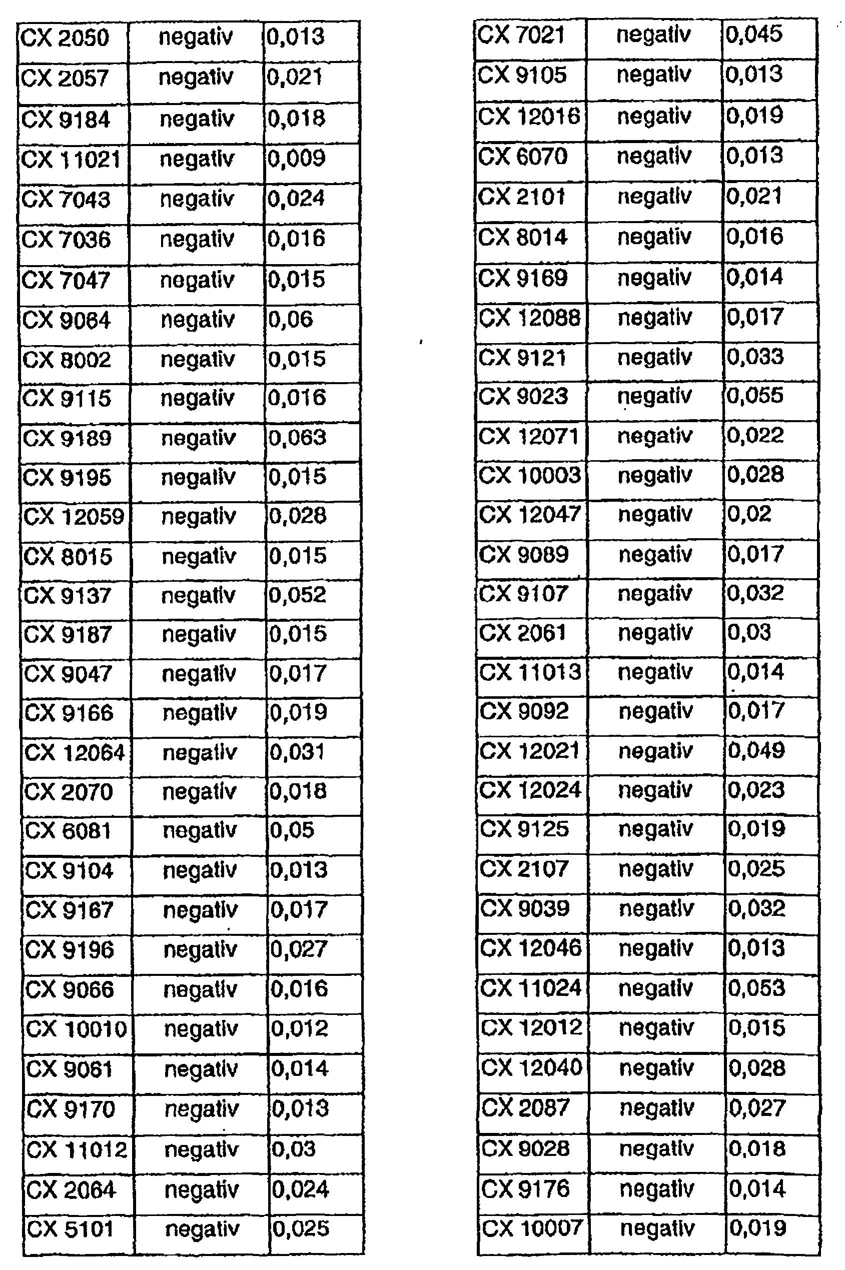

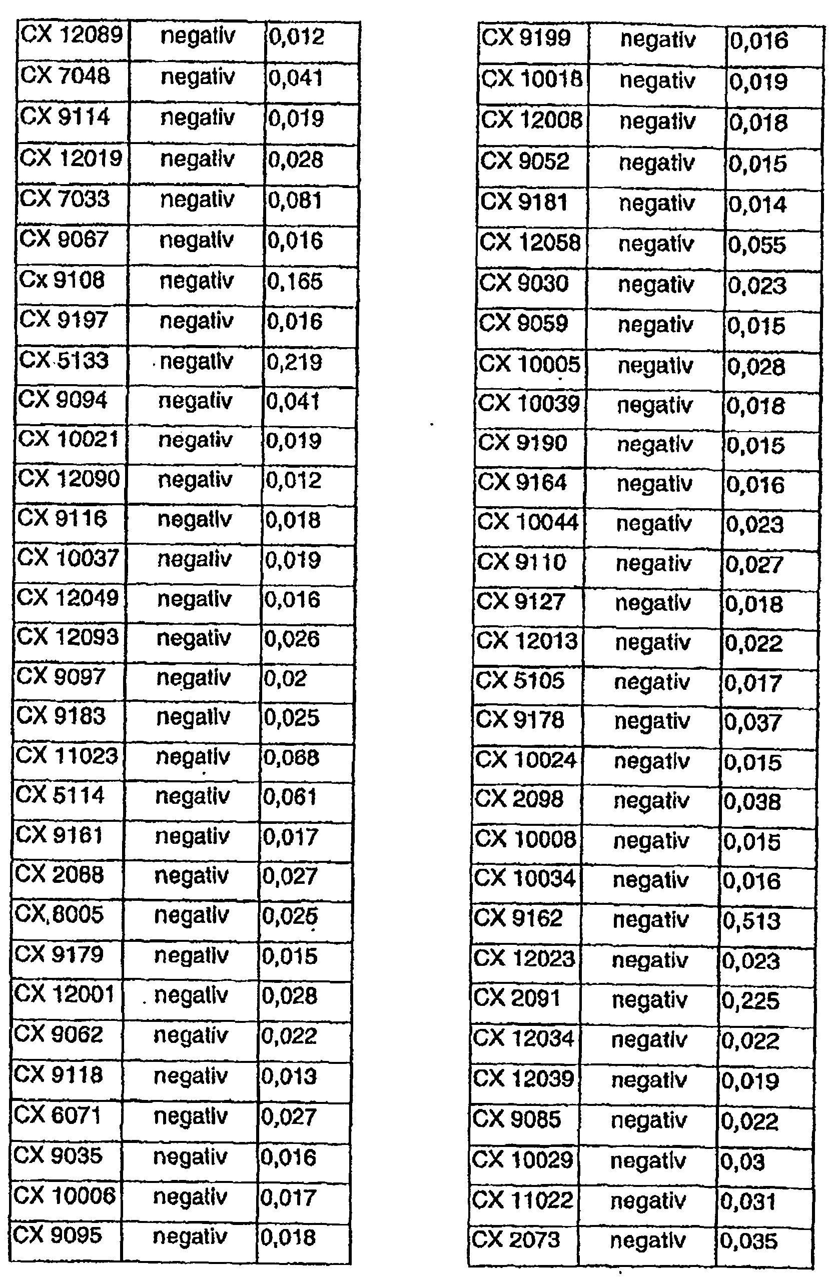

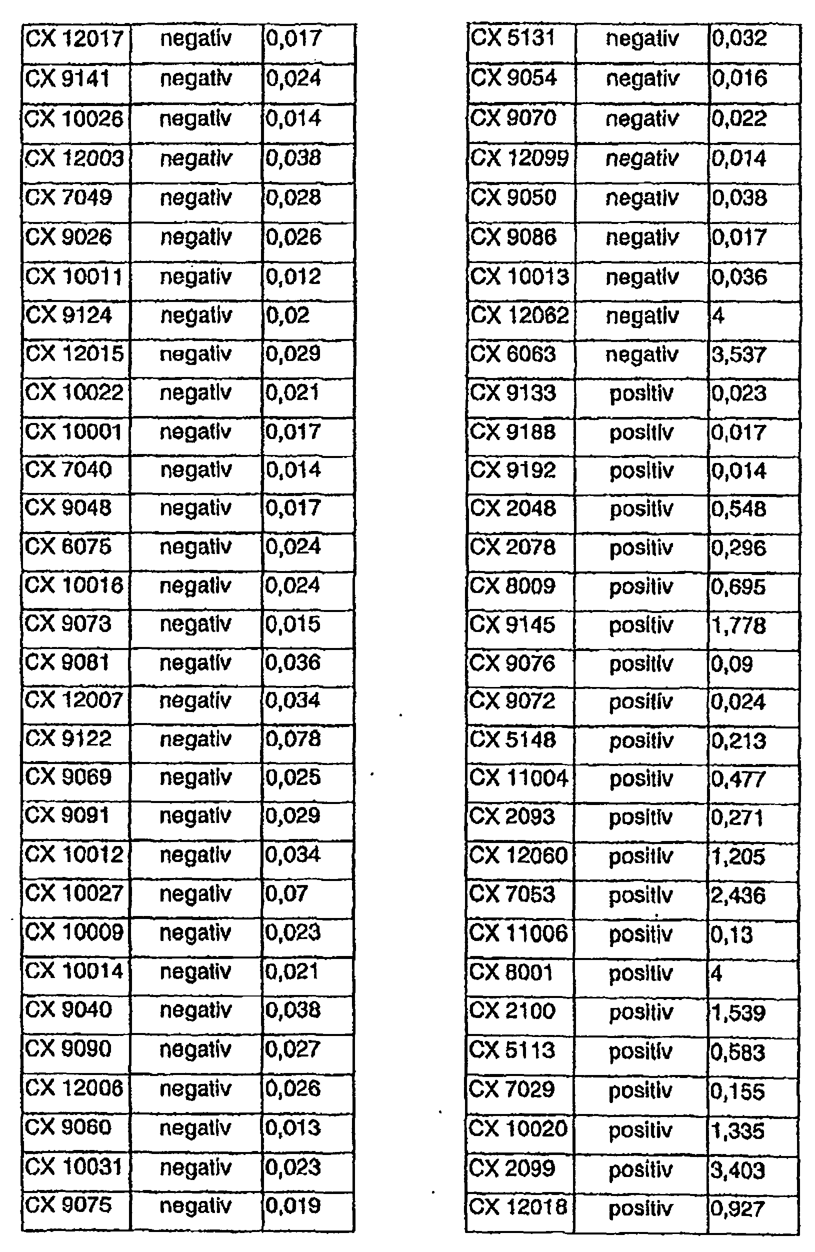

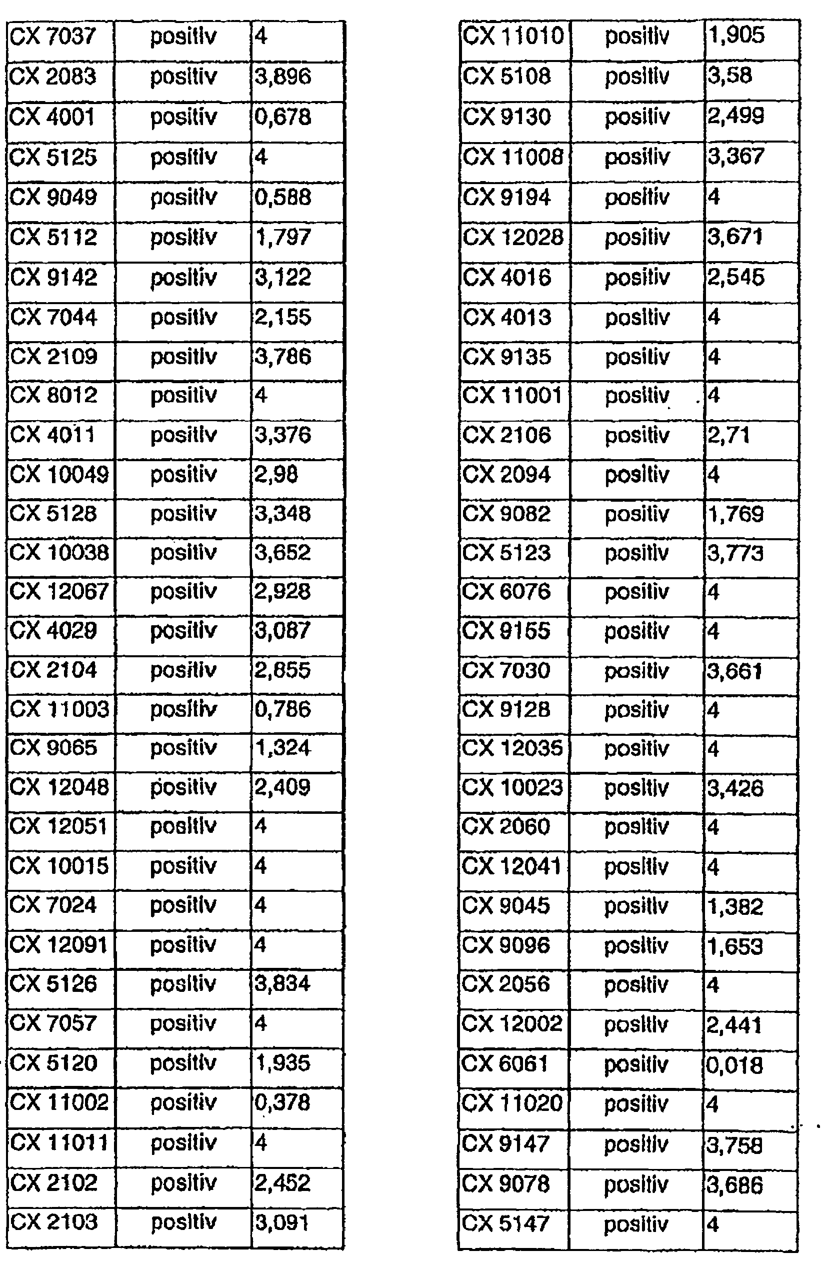

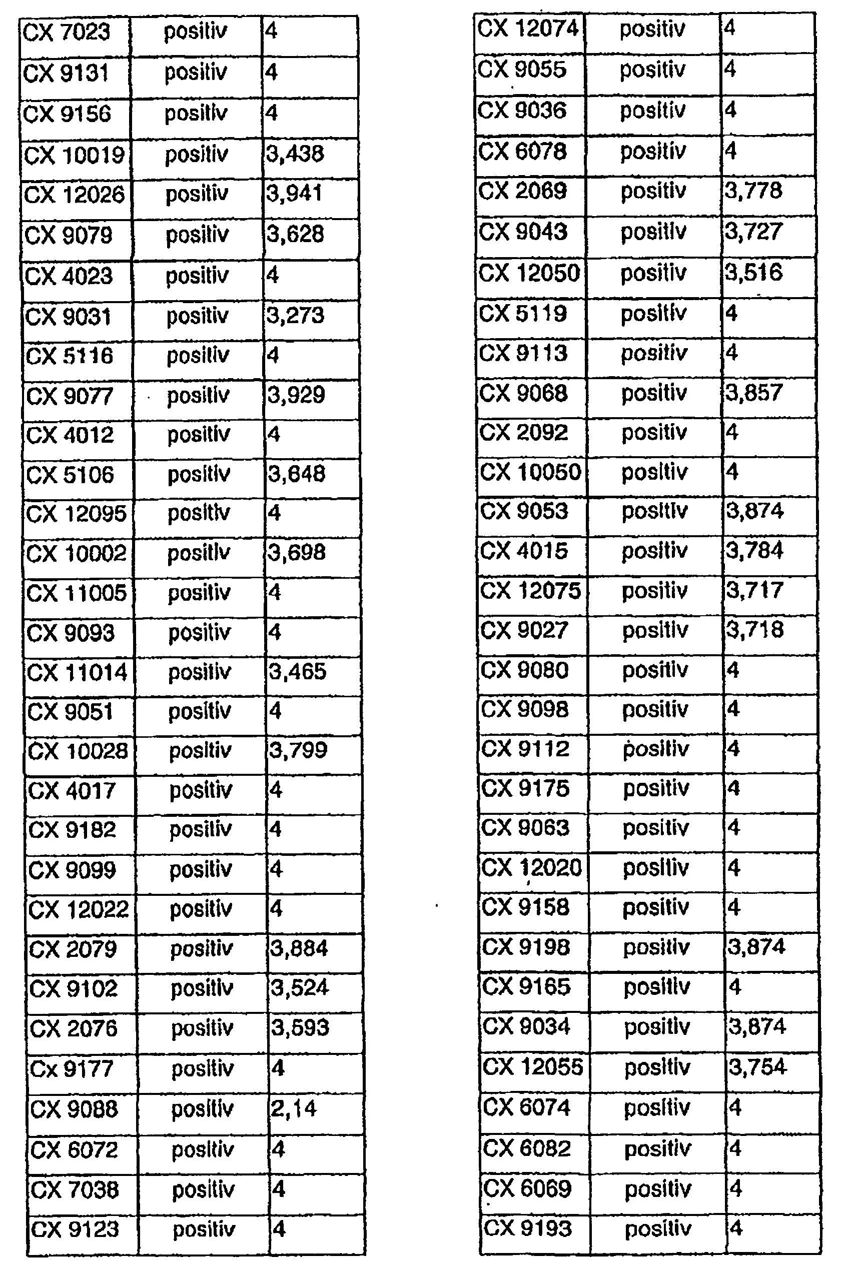

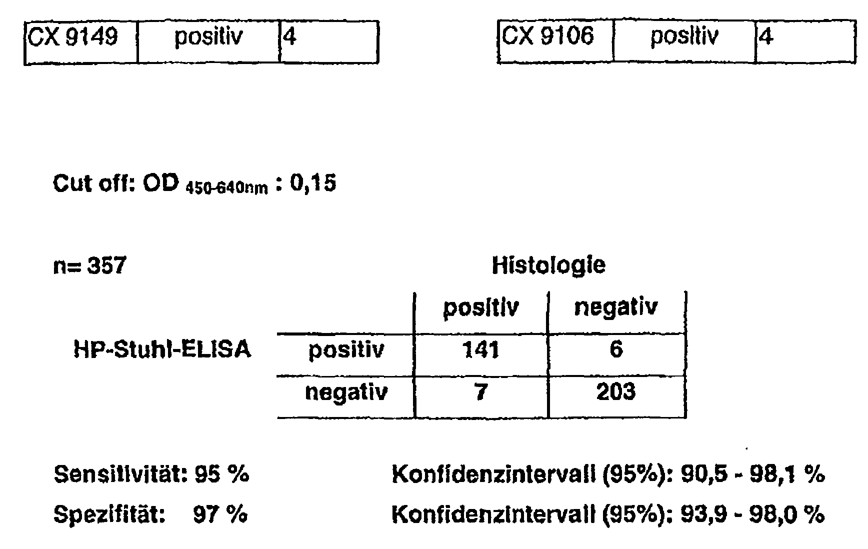

### Ergebnis:

Tabelle 8 zeigt die Ergebnisse der Untersuchung von *H*. *pylori*-negativen und *H*. *pylori-*positiven Stuhlproben (Erstdiagnose) mittels eines Stuhl-Sandwich-ELISA. Dabei wurden monoklonale Antikörper (Fang-Antikörper: HP25.6m/1B5; Detektionsantikörper HP25.2m/2H10-POD) für die Detektion des *H. pylori-*Antigens Katalase aus der Stuhlprobe eingesetzt. Die Untersuchung von Stuhlproben im rein monoklonalen ELISA-System, das nur auf Katalase-spezifische mAK aufgebaut ist, weist eine Sensitivität von 95 % und eine Spezifität von 97% auf.

### Beispiel 14: Eradikationsverlauf / Eradikationskontrolle

Eine Eradikationskontrolle ist nur über einen direkten Nachweis von *H. pylori*-Antigenen und nicht von Antikörper im Serum möglich, da *H*. *pylori*-Antikörper nach einer Infektion noch über viele Monate im Blut präsent sind. Daher bietet der dargestellte Sandwich-Stuhl-ELISA im Gegensatz zu serologischen *H. pylori*-Tests die Möglichkeit, den Erfolg einer Eradikation zu beurteilen.

In Figur 5 wird der Verlauf einer Eradikationsbehandlung eines *H. pylori*-positiven Patienten nach Einnahme von Omeprazol, Metronidazol und Clarithromycin gezeigt. 6 Tage nach Beginn der Behandlung konnte kein *H. pylori-*Antigen mehr im Stuhl nachgewiesen werden.

Tabelle 9 zeigt die Ergebnisse des HP-Stuhl ELISAs einer Eradikationsstudie. Die Stuhlproben wurden 4 bis 6 Wochen nach Beendigung der Eradikationstherapie gesammelt. Als Referenztest diente der ¹³C-Atemtest.
Die Testdurchführung erfolgte gemäß Beispiel 12 (Einschritt-ELISA).

**Tabelle 9: Eradikationskontrolle - Nachweis von H, pylori - Katalase aus Stuhl mittels Einschtitt-ELISA. Probennahme 4-6 Wochen nach Eradikationstherapie**

| **Patienten -Nr.** | **¹³C-Atemtest** | **HP-Stuhl-ELISA OD ₄₅₀₋₅₃₀ₙₘ** |
|---|---|---|
| 131 | negativ | 0,024 |
| 132 | negativ | 0,012 |
| 138 | negativ | 0,024 |
| 147 | negativ | 0,016 |
| 148 | negativ | 0,014 |
| 149 | negativ | 0,019 |
| 151 | negativ | 0,018 |
| 154 | negativ | 0,012 |
| 155 | negativ | 0,011 |
| 158 | negativ | 0.013 |
| 159 | negativ | 0,023 |
| 161 | negativ | 0.025 |
| 165 | negativ | 0,013 |
| 166 | negativ | 0,014 |
| 167 | negativ | 0,183 |
| 168 | negativ | 0.016 |
| 172 | negativ | 0.015 |
| 177 | negativ | 0,015 |
| 180 | negativ | 0.146 |
| 182 | negativ | 0,026 |
| 187 | negativ | 0,014 |
| 188 | negativ | 0,017 |
| 194 | negativ | 0,020 |
| 195 | negativ | 0,015 |
| 199 | nagativ | 0,013 |
| 205 | negativ | 0,035 |
| 206 | negativ | 0,020 |
| 213 | negativ | 0,018 |
| 215 | negativ | 0,014 |
| 216 | negativ | 0,034 |
| 217 | negativ | 0,014 |
| 219 | negativ | 0,014 |
| 223 | negativ | 0,086 |
| 224 | negativ | 0,020 |
| 227 | negativ | 0,139 |
| 245 | negativ | 0,094 |
| 246 | negativ | 0.120 |
| 250 | negativ | 0.019 |
| 251 | negativ | 0,042 |
| 253 | negativ | 0,034 |
| 265 | negativ | 0,033 |
| 256 | negativ | 0,041 |
| 270 | negativ | 0,053 |
| 271 | negativ | 0,033 |
| 275 | negativ | 0,040 |
| 283 | negativ | 0,036 |
| 284 | negativ | 0,018 |
| 296 | negativ | 0,170 |
| 303 | negativ | 0,064 |
| 308 | negativ | 0,029 |
| 310 | negativ | 0,025 |
| 311 | negativ | 0,013 |
| 312 | negativ | 0,049 |
| 315 | negativ | 0,021 |
| 318 | negativ | 0,037 |
| 319 | negativ | 0,044 |
| 320 | negativ | 0,057 |
| 322 | negativ | 0,019 |
| 324 | negativ | 0,017 |
| 326 | negativ | 0,154 |
| 327 | negativ | 0.016 |
| 328 | negativ | 0,015 |
| 329 | negativ | 0,266 |
| 330 | negativ | 0,035 |
| 331 | negativ | 0,013 |
| 337 | negativ | 0,015 |
| 338 | negativ | 0,051 |
| 339 | negativ | 0.021 |
| 350 | negativ | 0,037 |
| 353 | negativ | 0,019 |
| 356 | negativ | 0,023 |
| 357 | negativ | 0,025 |
| 358 | negativ | 0,057 |
| 359 | negativ | 0,023 |
| 360 | negativ | 0,073 |
| 366 | negativ | 0,018 |
| 367 | negativ | 0,018 |
| 368 | negativ | 0,029 |
| 369 | negativ | 0,028 |
| 152 | positiv | 0,365 |
| 156 | positiv | 0,264 |
| 160 | positiv | 3,851 |
| 162 | positiv | 2,021 |
| 169 | positiv | 0,112 |
| 179 | positiv | 0,573 |
| 181 | positiv | 2,886 |
| 186 | positiv | 2,084 |
| 196 | positiv | 0,282 |
| 220 | positiv | 0.905 |
| 240 | positiv | 2,837 |
| 252 | positiv | 1,606 |
| 278 | positiv | 3,173 |
| 300 | positiv | 0,840 |
| 325 | positiv | 3,898 |
| 334 | positiv | 2,946 |
| 361 | positiv | 0,269 |
| 161/1799 | positiv | 0,263 |

Im Vergleich zum Referenztest ergibt die Studie (97 Patienten) eine Sensitivität von 94% und eine Spezifität von 95% (cut-off: OD ₄₅₀₋₆₃₀: 0,15)

Beispiel 14 zeigt, daß der HP-Stuhl-ELISA nicht nur zum Nachweis einer *H*. *pylori* Erstdiagnose, sondern auch zur Eradikationskontrolle und zur Dokumentation des Eradikationsverlaufes geeignet ist.

### Beispiel 15: Klonierung und Sequenzbestimmung der funktionellen variablen Bereiche von Immunoglobulinen aus Hybridom-Zellinien

Gesamt-RNA wurde aus Antikörper-produzierenden Hybridom-Zellinien nach Chomczynski (Chomczynski, 1987) isoliert. Die entsprechende cDNA wurde nach Standard-Methoden synthetisiert (Sambrook et al., 1989)
Die DNA-Regionen, welche die kappa leichte Kette sowie das schwere Kette-Fd-Segment (VH bzw. CH1) der jeweiligen Antikörper kodieren, wurden mittels PCR amplifiziert. Dabei kam das in Tabelle 10 aufgeführte Oligunukleotid-Primer Set zur Anwendung. Die aus den einzelnen Hybridom-Zellinien isolierte cDNA diente als Matritze.
Das eingesetzte Primer-Set führt zu je einer 5'-*Xho* I und einer *3'-Spe* I Schnittstelle in den schwere Kette-Fd-Fragmenten sowie je einer 5'-*Sac* I und einer 3'*-Xba* I-Schnittstelle in den kappa leichten Ketten. Zur PCR-Amplifikation der schwere Kette-Fd-kodierenden DNA-Fragmente wurden 11 verschiedene 5'-VH-Primer (MVH 1-8 und MULH1-3) jeweils kombiniert mit dem 3'-VH-Primer MIgG2a (HP25.2m/2H10) bzw. mit dem 3'-VH-Primer MIgG1 (HP25.6m/1B5) verwendet. Zur Amplifikation der DNA-Fragmente, welche für die kappa leichten Ketten codieren, wurden 11 verschiedene 5'-VK-Primer (MUVK 1-7 und MULK1-4) jeweils mit dem 3'-VK-Primer 3'MUCK kombiniert.
Das folgende Temperaturprogramm kam bei allen PCR-Amplifikationen zur Anwendung: Denaturierung bei 94°C für 30 s, Primer-Anlagerung bei 52°C für 60 s, Polymerisation bei 72°C für 90 s. Dieses Programm wurde für 40 Zyklen beibehalten, gefolgt von einer 10 min abschließenden Vervollständigung der Fragmente bei 72°C.
Die Ergebnisse der PCR-Amplifikationen wurden mittels Agarose-Gel-Elektrophorese aufgetrennt und DNA-Banden des erwarteten Molekulargewichts isoliert.
Für den Antikörper 25.2m/2H10 wurden die isolierten Banden anschließend einem Restriktionsverdau unter Einsatz der Enzyme *Xho* I und *Spe* I (schwere Ketten) bzw. *Sac* I und *Xba* I (leichte Ketten) unterzogen und die erhaltenenen Fragmente in den Plasmid-Vektor Bluescript KS (Stratagene) kloniert, nachdem dieser zunächst mit den Restriktionsenzymen *Xho* I und *Spe* I bzw. *Sac* I und *Xba* I gespalten worden war.
Plasmid-Präparationen der klonierten schwere und leichte Ketten-Fragmente wurden daraufhin sequenzanalysiert. Es wurden Sequenzen ausgewählt, die für funktionelle variable Bereiche der Immunglobulin schwere und leichte Kette (VH bzw. VL) codieren. Auf diese Weise konnte für diese Hybridom-Zellinie genau ein funktioneller VH- und ein funktioneller VL-Bereich identifiziert werden. Die funktionellen VH- und VL-Sequenzen sind in Fig. 1/Fig. 2 wiedergegeben. Die ersten vier Aminosäuren der VH-Region wurden durch Umklonierung ergänzt. Klonierung und Sequenzierung wurden nach Standardmethoden durchgeführt (Sambrook et al., 1989).

Für den Antikörper HP25.6m/1B5 wurden die isolierten Banden anschließend direkt sequenziert und eine funktionelle leichte und eine funktionelle schwere Kette identifiziert. Das schwere-Ketten-Fd-Fragment und die leichte Kette wurden anschließend einem Restriktionsverdau unter Einsatz der Enzyme Xhol und Spel (Schwere Kette) bzw. Sacl und Xbal (Leichte Kette) unterzogen und die erhaltenenen Fragmente in den Plasmid-Vektor pBSIIIHisEx (Connex) kloniert, nachdem dieser mit den Restriktionsenzymen *Xho* I und *Spe* I bzw. *Sac* I und *Xba* I gespalten worden war, und erneut sequenziert.

Auf diese Weise konnte für diese Hybridom-Zellinie genau ein funktioneller VH- und ein funktioneller VL-Bereich identifiziert werden. Die funktionellen VH- und VL-Sequenzen sind in Fig. 3/Fig. 4 wiedergegeben. Bei den VH- und VL Sequenzen sind die reifen N-Termini dargestellt, wie sie durch die Sequenzierung mittels Leaderprimer ermittelt wurden. Klonierung und Sequenzierung wurden nach Standardmethoden durchgeführt (Sambrook et al., 1989).

**Tabelle 10: Liste der für die PCR-Ampliflkation der funktionellen variablen Bereiche von schweren und leichten Immunglobulin-Ketten verwendeten Primer (Orientierung 5' - 3')**

| | |
|---|---|
| MVH1 | (GC)AG GTG CAG CTC GAG GAG TCA GGA CCT |
| MVH2 | GAG GTC CAG CTC GAG CAG TCT GGA CCT |
| MVH3 | CAG GTC CAA CTC GAG CAG CCT GGG GCT |
| MVH4 | GAG GTT CAG CTC GAG CAG TCT GGG GCA |
| MVH5 | GA(AG) GTG AAG CTC GAG GAG TCT GGA GGA |
| MVH6 | GAG GTG AAG CTT CTC GAG TCT GGA GGT |
| MVH7 | GAA GTG AAG CTC GAG GAG TCT GGG GGA |
| MVH8 | GAG CTT CAG CTC GAG CAG TCT GGA GCT |
| MULK1 | GGG GAG CTC CAC CAT GGA GAC AGA CAC ACT CCT GCT AT |
| MULK2 | GGG GAG CTC CAC CAT GGA TTT TCA AGT GCA GAT TTT CAG |
| MULK3 | GGG GAG CTC CAC CAT GGA GWC ACA KWC TCA GGT CTT TRT A |
| MULK4 | GGG GAG CTC CAC CAT GKC CCC WRC TCA GYT YCT KGT |
| MIgG1 | TAT GCA ACT AGT ACA ACC ACA ATC CCT GGG |
| MlgG2a | GAG AGA GGG GTT CTG ACT AGT GGG CAC TCT GGG CTC |
| MUVK1 | CCA CTT CCG AGC TCG TTG TGA CTC AGG AAT CT |
| MUVK2 | CCA GTT CCG AGC TCG TGT TGA CGC AGC CGC CC |
| MUVK3 | CCA GTT CCG AGC TCG TGC TCA CCC AGT CTC CA |
| MUVK4 | CCA GTT CCG AGC TCC AGA TGA CCC AGT CTC CA |
| MUVK5 | CCA GAT GTG AGC TCG TGA TGA CCC AGA CTC CA |
| MUVK6 | CCA GAT CTG AGC TCG TCA TGA CCC AGT CTC CA |
| MUVK7 | CCA GTT CCG AGC TCG TGA TGA CAC AGT CTC CA |
| MULH1 | GGG CTC GAG CAC CAT GGR ATG SAG CTG KGT MAT SCT CTT |
| MULH2 | GGG CTC GAG CAC CAT GRA CTT CGG GYT GAG CTK GGT TTT |
| MULH3 | GGG CTC GAG CAC CAT GGC TGT CTT GGG GCT GCT CTT CT |
| 3'MUCK | GCG CCG TCT AGA ATT AAC ACT CAT TCC TGT TGA A |

### Literatur:

Coller & Coller, 1983: Coller, H.A., Coller, B.S., Meth. Enzymol. 121:412-417
Harlow & Lane, 1988: Harlow, E., Lane, D., Antibodies: A laboratory manual, Cold Spring Harbour Laboratory, New York
Kearney et al., 1979: Kearney, J. Immunol. 123: 1548-1550
Laemmli, 1970: Laemmli, E.K., Nature 227: 680-685
Peters & Baumgarten, 1990: Peters, J.H., Baumgarten, H. (Hrsg.), Monoklonale Antikörper, Springer Verlag, Berlin
Fägerstam et al., 1990: Fägerstam, L.G. et al., J. Mol. Recognit. 3: 208-214
Malmqvist, 1996: Methods 9: 525-532
Eschweiler et al., 1993: Eschweiler, B., et al., Zentralbl. F. Bakt. 280: 73-85
Pharmacia Biotech, 1994: Monoclonal Antibody Purification Handbook
Chomczynski, 1987: Anal. Biochem. 162: 156-159
Sambrook et al., 1989: Molecular cloning: A laboratory manual, Cold Spring Harbour Laboratory Press, second edition
Vaughan et al., 1998: Nature Biotechnology 16: 535-539
Orlandi et al., 1989: Proc. Natl. Acad. Sci USA 86: 3833-3837
Janeway & Travers, 1997: Immunologie, 2. Auflage, Spektrum Akademischer
Verlag GmbH, Heidelberg
Osborne et al., 1997: Curr. Opin. Chem. Biol. 1: 5-9
Stull und Szoka, 1995: Pharm. Res. 12: 465-483
Frens, 1973: Nat. Phys. Sci. 241, 20-23
Geoghegan and Ackerman, 1977: J. of Histochemistry and Cytochemistry, 25(11), 1187-1200
Slot, 1985: Eur. J. Cell Biol. 38, 87-93
Manos et al., 1998: Helicobacter 3 (1), 28-38
Haque, 1993: J. Infect. Dis. 167: 247-9
Park, 1996: J. Clin. Microbiol. 34: 988-990
Hasan, 1994: FEMS Microbiol. Lett. 120: 143-148
Koopmans, 1993: J. Clin. Microbiol. 31: 2738-2744
Machnicka,1996: Appl. Parasitol. 37: 106-110

## Patentansprüche

1. Verfahren zum Nachweis einer Infektion eines Säugers mit einem Säure-resistenten Bakterium der Gattung Helicobacter, wobei man
(a) eine Stuhlprobe des Säugers unter Verwendung (aa) eines Rezeptors unter Bedingungen inkubiert, die eine Komplexbildung eines Antigens aus dem Säure-resistenten Bakterium mit dem Rezeptor erlauben; oder (ab) zwei unterschiedliche Rezeptoren unter Bedingungen inkubiert, die eine Komplexbildung eines Antigens aus dem Säure-resistenten Bakterium mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Bakterium oder einem Extrakt oder Lysat davon oder einem Protein daraus oder einem Fragment davon oder einem synthetischen Peptid Antikörper produziert; und
(b) die Bildung des einen Antigen-Rezeptorkomplexes gemäß (a) nachweist,
wobei
das Antigen ein Antigen einer Katalase ist und
der Nachweis der Bildung des einen Antigen-Rezeptor-Komplexes in Schritt (b) mittels ELISA, RIA, Western Blot oder eines immunchromatographischen Verfahrens erfolgt.

2. Verfahren nach Anspruch 1, wobei das Bakterium ein Bakterium der Spezies *Helicobacter pylori* oder *Helicobacter hepaticus* ist.

3. Verfahren nach Anspruch 2, wobei das Antigen ein Antigen von H. pylori ist.

4. Verfahren nach einem der Ansprüche 1 bis 3, wobei der Rezeptor/die Rezeptoren (ein) Antikörper, (ein) Fragment(e) oder Derivat(e) davon ist/sind.

5. Verfahren nach einem der Ansprüche 1 bis 4, wobei für den Nachweis zusätzlich ein Gemisch von Rezeptoren eingesetzt wird, wobei das Gemisch von Rezeptoren als Fänger des Antigens fungiert, wenn der Rezeptor als Detektor des Antigens eingesetzt wird und das Gemisch als Detektor des Antigens fungiert, wenn der Rezeptor als Fänger des Antigens eingesetzt wird.

6. Verfahren nach Anspruch 5, wobei das Gemisch von Rezeptoren ein polyklonales Antiserum ist.

7. Verfahren nach Anspruch 6, wobei das polyklonale Antiserum gegen ein Lysat des Bakteriums gewonnen wurde.

8. Verfahren nach Anspruch 7, wobei das Lysat ein Lysat mit angereichertem Antigen ist.

9. Verfahren nach Anspruch 7 oder 8, wobei das Lysat ein Lysat mit abgereicherten immundominanten Antigenen ist.

10. Verfahren nach Anspruch 8, wobei das polyklonale Antiserum gegen ein aufgereinigtes oder ein (semi)synthetisch hergestelltes Antigen gewonnen wurde.

11. Verfahren nach einem der Ansprüche 1 bis 10, wobei der Rezeptor und/oder das Gemisch von Rezeptoren (ein) Konformationsepitop(e) bindet/n.

12. Verfahren nach einem der Ansprüche 4 bis 11, wobei die schwere Kette des ein Katalase-Epitop bindenden Antikörpers mindestens eine der folgenden CDRs, und vorzugsweise die CDR3 aufweist:
| | |
|---|---|
| CDR1: | NYWIH |
| CDR2: | YINPATGSTSYNQDFQD |
| CDR3: | EGYDGFDS |

13. Verfahren nach Anspruch 12, wobei die schwere Kette alle drei genannten CDRs aufweist.

14. Verfahren nach Anspruch 12 oder 13, wobei die die schwere Kette des Antikörpers kodierende DNA-Sequenz mindestens eine der folgenden CDRs, vorzugsweise die CDR3 und weiter bevorzugt alle drei folgenden CDRs aufweist:
| | |
|---|---|
| CDR1: | AACTACTGGA TTCAC |
| CDR2: | TACATTAATC CTGCCACTGG TTCCACTTCT TACAATCAGG ACTTTCAGGA C |
| CDR3: | GAGGGGTACG ACGGGTTTGA CTCC |

15. Verfahren nach einem der Ansprüche 4 bis 11, wobei die leichte Kette des eine Katalase-Epitop bindenden Antikörpers mindestens eine der folgenden CDRs, vorzugsweise die CDR3 und weiter bevorzugt alle drei folgenden CDRs aufweist:
| | |
|---|---|
| CDR1: | SASSSVNYMY |
| CDR2: | DTSKLAS |
| CDR3: | QQWSSNPYT |

16. Verfahren nach Anspruch 15, wobei die die leichte Kette des Antikörpers kodierende DNA-Sequenz mindestens eine der folgenden CDRs, vorzugsweise die CDR3 und weiter bevorzugt alle drei folgenden CDRs aufweist:
| | |
|---|---|
| CDR1: | AGTGCCAGCT CAAGTGTAAA TTACATGTAC |
| CDR2: | GACACATCCA AATTGGCTTC T |
| CDR3: | CAGCAGTGGA GTAGTAATCC GTACACG |

17. Verfahren nach einem der Ansprüche 4 bis 11, wobei die schwere Kette des ein Katalase-Epitop bindenden Antikörpers mindestens eine der folgenden CDRs, vorzugsweise die CDR3 und weiter bevorzugt alle drei folgenden CDRs aufweist:
| | |
|---|---|
| CDR1: | DTYVH |
| CDR2: | KIDPANGKTKYDPIFQA |
| CDR3: | PIYYASSWFAY |

18. Verfahren nach Anspruch 17, wobei die die schwere Kette des Antikörpers kodierende DNA-Sequenz mindestens eine der folgenden CDRs, vorzugsweise die CDR3 und weiter bevorzugt alle drei folgenden CDRs aufweist:
| | |
|---|---|
| CDR1: | GACACCTATGTGCAC |
| CDR2: | AAGATTGATCCTGCGAATGGTAAAACTAAATAT GACCCGATATTCCAGGCC |
| CDR3: | CCCATTTATTACGCTAGTTCCTGGTTTGCTTAC |

19. Verfahren nach einem der Ansprüche 4 bis 11, wobei die die leichte Kette des ein Katalase-Epitop bindenden Antikörpers mindestens eine der folgenden CDRs, vorzugsweise die CDR3 und weiter bevorzugt alle drei folgenden CDRs aufweist:
| | |
|---|---|
| CDR1: | KASQDVGTSVA |
| CDR2: | WTSTRHT |
| CDR3: | QYSSSPT |

20. Verfahren nach Anspruch 19, wobei die die leichte Kette des Antikörpers kodierende DNA-Sequenz mindestens eine der folgenden CDRs, vorzugsweise die CDR3 und weiter bevorzugt alle drei folgenden CDRs aufweist:
| | |
|---|---|
| CDR1: | AAGGCCAGTCAGGATGTGGGTACTTCTGTTGCC |
| CDR2: | TGGACATCCACCCGGCACACT |
| CDR3: | CAGCAATATAGCAGCTCTCCCACG |

21. Verfahren nach einem der Ansprüche 4 bis 20, wobei die Antikörper in den variablen Regionen der leichten und schweren Ketten die in den Figuren 1 und 2, 3 und 4 dargestellten Aminosäuresequenzen aufweisen.

22. Verfahren nach einem der Ansprüche 4 bis 21, wobei die kodierenden Bereiche der variablen Regionen der leichten und schweren Ketten die in den Figuren 1 und 2, 3 und 4 dargestellten DNA-Sequenzen aufweisen.

23. Verfahren nach einem der Ansprüche 1 bis 22, wobei mit der Stuhlprobe vor der Inkubation mit den Antikörpern folgende Schritte durchgeführt werden:
(a) Resuspendieren der Stuhlprobe 1:3 bis 1:25, vorzugsweise etwa 1:5 bis 1:10, besonders bevorzugt 1:5 in Resuspendierungspuffer und
(b) Mischen auf einem Vortexmixer.

24. Verfahren nach einem der Ansprüche 1 bis 23, wobei im RIA oder im ELISA der gleiche Rezeptor zur Bindung an die Festphase wie zum Nachweis des Epitops eingesetzt wird.

25. Verfahren nach einem der Ansprüche 1 bis 24, wobei der Rezeptor an einen Träger fixiert ist.

26. Verfahren nach einem der Ansprüche 1 bis 25 wobei der Rezeptor ein monoklonaler Maus-Antikörper ist.

27. Verfahren nach einem der Ansprüche 1 bis 26, bei dem das Verfahren ein Einschritt-ELISA ist.

28. Verfahren nach einem der Ansprüche 1 bis 26, bei dem das Verfahren ein Dreischritt-ELISA ist.

29. Verfahren nach Anspruch 25, wobei das Trägermaterial des Trägers ein poröses Trägermaterial ist.

30. Verfahren nach Anspruch 25 oder 29, wobei das Trägermaterial ein Teststreifen ist.

31. Verfahren nach Anspruch 25, 29 oder 30, wobei das Trägermaterial aus Zellulose oder einem Zellulosederivat besteht.

32. Verfahren nach einem der Ansprüche 1 bis 31, bei dem anstelle einer Stuhlprobe Zahnplaque oder Speichel zum Nachweis eingesetzt wird.

33. Verfahren nach einem der Ansprüche 1 bis 32, bei dem es sich um ein automatisiertes Verfahren handelt.

34. Verfahren nach einem der Ansprüche 1 bis 33, wobei der Säuger ein Mensch ist.

35. Monoklonaler Antikörper, Fragment oder Derivat davon, der/das eine V-Region aufweist, die eine Kombination der in einem der Ansprüche 12 bis 20 dargestellten CDRs aufweist.

36. Monoklonaler Antikörper, Fragment oder Derivat davon nach Anspruch 35, der/das mindestens eine der in den Figuren 1 und 2 oder 3 und 4 dargestellten V-Regionen aufweist.

37. Monoklonaler Antikörper, Fragment oder Derivat davon nach Anspruch 35 oder 36, der ein Maus-Antikörper oder ein Fragment oder Derivat davon oder ein chimärer, vorzugsweise ein humanisierter Antikörper oder ein Fragment oder Derivat davon ist.

38. Verwendung einer diagnostischen Zusammensetzung enthaltend einen Rezeptor wie in einem der Ansprüche 1 bis 34, und 35 bis 37 definiert, der ein Antigen einer Helicobacter Katalase spezifisch bindet und der gegebenenfalls fixiert ist an ein Trägermaterial, zum Nachweis von Helicobacter im Stuhl.

39. Testvorrichtung zum Nachweis mindestens eines wie in einem der vorstehenden Ansprüchen definierten Epitops, umfassend:
(a) einen Rezeptor wie in einem der Ansprüche 1 bis 34, 35 bis 37 definiert, der ein Antigen einer Helicobacter Katalase spezifisch bindet, fixiert an ein Trägermaterial; und
(b) eine Vorrichtung zur Aufbereitung und Analyse von Stuhlproben.

40. Testvorrichtung zum Nachweis mindestens eines wie in einem der vorstehenden Ansprüche definierten Epitops, umfassend:
(a) einen Rezeptor wie in einem der Ansprüche 1 bis 34, 35 bis 37 definiert, der ein Antigen einer Helicobacter Katalase spezifisch bindet, wobei der Rezeptor konjugiert ist mit kolloidalem Gold, Latexpartikeln oder anderen farbgebenden Partikeln, deren Größe typischerweise im Bereich zwischen 5nm und 100nm, vorzugsweise zwischen 20nm und 60nm liegt; besonders bevorzugt zwischen 40nm und 60nm (Gold) bzw. 200 nm bis 500nm (Latex); und
(b) eine Vorrichtung zur Aufbereitung und Analyse von Stuhlproben.

41. Kit enthaltend
(a) einen Rezeptor wie in einem der Ansprüche 1 bis 34, 35 bis 37 definiert, der ein Antigen einer Helicobacter Katalase spezifisch bindet, gegebenenfalls fixiert an ein Trägermaterial; und gegebenenfalls ferner
(b) eine Vorrichtung zur Aufbereitung und Analyse von Stuhlproben.

42. Packung enthaltend die Testvorrichtung nach Anspruch 39 oder 40 oder den Kit nach Anspruch 41.

43. Verwendung eines einzigen Rezeptors, der ein Epitop eines Antigens eines Säure-resistenten Bakteriums der Gattung Helicobacter spezifisch bindet, oder von zwei Rezeptoren, die zwei Epitope desselben Antigens spezifisch binden, wobei das Antigen Helicobacter-Katalase ist, zum Nachweis der Infektion eines Säugers mit diesen Bakterien im Stuhl.

## Claims

1. A method for detecting an infection of a mammal with an acid-resistant bacterium belonging to the genus Helicobacter, wherein
(a) a stool sample of the mammal is incubated using (aa) a receptor under conditions allowing a complex formation of an antigen from the acid-resistant bacterium with the receptor; or (ab) two different receptors under conditions allowing a complex formation of an antigen from the acid-resistant bacterium with the two receptors and wherein the receptor according to (aa) or the receptors according to (ab) specifically bind(s) an antigen which shows, at least with some mammals, a structure after passage through the intestine that corresponds to the native structure or the structure which a mammal produces antibodies against after being infected or immunized with the acid-resistant bacterium or an extract or lysate thereof or a protein therefrom or a fragment thereof or a synthetic peptide; and
(b) the formation of the antigen-receptor complex according to (a) is detected,
wherein
the antigen is the antigen of a catalase and
the detection of the formation of the one antigen receptor complex in step (b) takes place by means of an ELISA, RIA, Westernblot or an immunochromatographic method.

2. The method according to claim 1, wherein the bacterium is a bacterium belonging to the species *Helicobacter pylori* or *Helicobacter hepaticus.*

3. The method according to claim 2, wherein the antigen is an antigen of H. pylori.

4. The method according to any one of claims 1 to 3, wherein the receptor/the receptors is (are) (an) antibody(ies), (a) fragment(s) or derivative(s) thereof.

5. The method according to any one of claims 1 to 4, wherein for the detection additionally a mixture of receptors is used, wherein the mixture of receptors has the function of catching the antigen if the receptor is used as detector of the antigen, and the mixture has the function of detecting the antigen if the receptor is used as catcher of the antigen.

6. The method of claim 5, wherein the mixture of receptors is a poly-clonal antiserum.

7. The method according to claim 6, wherein the polyclonal antiserum is obtained against a lysate of the bacterium.

8. The method according to claim 7, wherein the lysate is a lysate with enriched antigen.

9. The method according to claim 7 or 8, wherein the lysate is a lysate with depleted immunodominant antigens.

10. The method according to claim 8, wherein the polyclonal antiserum is obtained against a purified or a (semi-)synthetically produced antigen.

11. The method according to any one of claims 1 to 10, wherein the receptor and/or the mixture of receptors bind(s) (a) conformational epitope(s).

12. The method according to any one of claims 4 to 11, wherein the heavy chain of the antibody binding a catalase epitope has at least one of the following CDRs, preferably the CDR3:
| | |
|---|---|
| CDR1: | NYWIH |
| CDR2: | YINPATGSTSYNQDFQD |
| CDR3: | EGYDGFDS |

13. The method according to claim 12, wherein the heavy chain has all the three CDRs mentioned.

14. The method according to claim 12 or 13, wherein the DNA sequence encoding the heavy chain of the antibody has at least one of the following CDRs, preferably the CDR3 and still more preferably all of the three following CDRs:
| | |
|---|---|
| CDR1: | AACTACTGGA TTCAC |
| CDR2: | TACATTAATC CTGCCACTGG TTCCACTTCT TACAATCAGG ACTTTCAGGA C |
| CDR3: | GAGGGGTACG ACGGGTTTGA CTCC |

15. The method according to any one of claims 4 to 11, wherein the light chain of the antibody binding a catalase epitope has at least one of the following CDRs, preferably the CDR3 and still more preferably all of the three following CDRs:
| | |
|---|---|
| CDR1: | SASSSVNYMY |
| CDR2: | DTSKLAS |
| CDR3: | QQWSSNPYT |

16. The method according to claim 15, wherein the DNA sequence encoding the light chain of the antibody has at least one of the following CDRs, preferably the CDR3 and still more preferably all of the three following CDRs:
| | |
|---|---|
| CDR1: | AGTGCCAGCTCAAGTGTAAATTACATGTAC |
| CDR2: | GACACATCCA AATTGGCTTC T |
| CDR3: | CAGCAGTGGA GTAGTAATCC GTACACG |

17. The method according to any one of claims 4 to 11, wherein the heavy chain of the antibody binding a catalase epitope has at least one of the following CDRs, preferably the CDR3 and more preferably all three of the following CDRs:
| | |
|---|---|
| CDR1: | DTYVH |
| CDR2: | KIDPANGKTKYDPIFQA |
| CDR3: | PIYYASSWFAY |

18. The method according to claim 17, wherein the DNA sequence encoding the heavy chain of the antibody has at least one of the following CDRs, preferably CDR3 and more preferably all three of the following CDRs:
| | |
|---|---|
| CDR1: | GACACCTATGTGCAC |
| CDR2: | AAGATTGATCCTGCGAATGGTAAAACTAAATAT GACCC GATATTCCAGGCC |
| CDR3: | CCCATTTATTACGCTAGTTCCTGGTTTGCTTAC |

19. The method according to any one of claims 4 to 11, wherein the light chain of the antibody binding a catalase epitope has at least one of the following CDRs, preferably CDR3 and more preferably all three of the following CDRs:
| | |
|---|---|
| CDR1: | KASQDVGTSVA |
| CDR2: | WTSTRHT |
| CDR3: | QYSSSPT |

20. The method according to claim 19, wherein the DNA sequence encoding the light chain of the antibody has at least one of the following CDRs, preferably CDR3 and more preferably all three of the following CDRs:
| | |
|---|---|
| CDR1: | AAGGCCAGTCAGGATGTGGGTACTTCTGTTGCC |
| CDR2: | TGGACATCCACCCGGCACACT |
| CDR3: | CAGCAATATAGCAGCTCTCCCACG |

21. The method according to any one of claims 4 to 20, wherein the antibodies in the variable regions of the light and heavy chains have the amino acid sequences shown in Figures 1 and 2, 3 and 4.

22. The method according to any one of claims 4 to 21, wherein the coding regions of the variable regions of the light and heavy chains have the DNA sequences shown in Figures 1 and 2, 3 and 4.

23. The method according to any one of claims 1 to 22, wherein the following steps are carried out with the stool sample before incubation with the antibodies:
(a) resuspending the stool sample at a ratio of 1:3 to 1:25, preferably approximately at a ratio of 1:5 to 1:10, particularly preferably 1:5, in resuspension buffer and
(b) mixing on a vortex mixer.

24. The method according to any one of claims 1 to 23, wherein in the RIA or in the ELISA the same receptor is used for both binding to the solid phase and detecting the epitope.

25. The method according to any one of claims 1 to 24, wherein the receptor is fixed to a support.

26. The method according to any one of claims 1 to 25, wherein the receptor is a monoclonal murine antibody.

27. The method according to any one of claims 1 to 26, wherein the method is a one-step ELISA.

28. The method according to any one of claims 1 to 26, wherein the method is a three-step ELISA.

29. The method according to claim 25, wherein the material of the support is a porous material.

30. The method according to claim 25 or 29, wherein the material of the support is a test strip.

31. The method according to claims 25, 29 or 30, wherein the material of the support consists of cellulose or a cellulose derivative.

32. The method according to any one of claims 1 to 31, wherein tooth plaque or saliva is used for the detection instead of a stool sample.

33. The method according to any one of claims 1 to 32, wherein the method is a automated method.

34. The method according to any one of claims 1 to 33, wherein the mammal is a human.

35. A monoclonal antibody, fragment or derivative thereof which has a V region that shows a combination of the CDRs illustrated in any one of claims 12 to 20.

36. The monoclonal antibody, fragment or derivative thereof according to claim 35 which has at least one of the V regions shown in Figures 1 and 2 or 3 and 4.

37. The monoclonal antibody, fragment or derivative thereof according to claim 35 or 36 which is a murine antibody or a fragment or derivative thereof or a chimeric, preferably a humanized antibody or a fragment or derivative thereof.

38. Use of a diagnostic composition comprising a receptor as defined in anyone of claims 1 to 34 and 35 to 37, which specifically binds an antigen of a catalase of Helicobacter, and is optionally fixed to a support material, to detect Helicobacter in the stool.

39. A test device for the detection of at least one of the epitopes as defined in any one of the preceding claims, comprising:
(a) a receptor as defined in any one of claims 1 to 34, 35 to 37, which specifically binds an antigen of a catalase of Helicobacter, fixed to a support material; and
(b) a device for preparing and analyzing stool samples.

40. A test device for the detection of at least one epitope as defined in any one of the preceding claims, comprising
(a) at least one receptor as defined in any one of claims 1 to 34, 35 to 37, which specifically binds an antigen of a catalase of Helicobacter and wherein the receptor is conjugated with colloidal gold, latex particles or other coloring particles, the size of which typically ranges between 5 nm and 100 nm, preferably between 20 nm and 60 nm, particularly preferably between 40 nm and 60 nm (gold) and 200 nm and 500 nm (latex) respectively; and
(b) a device for preparing and analyzing stool samples.

41. A kit containing
(a) a receptor as defined in any one of claims 1 to 34, 35 to 37, which specifically binds an antigen of a catalase of Helicobacter optionally fixed to a support; and optionally furthermore
(b) a device for preparing and analyzing stool samples.

42. A package containing the test device according to claim 39 or 40 or the kit according to claim 41.

43. Use of a single receptor specifically binding an epitope of an antigen of an acid-resistant bacterium of the genus Helicobacter, or use of two receptors, specifically binding two epitopes of the same antigen, wherein the antigen is a catalase of Helicobacter, for detecting in the stool an infection of a mammal with said bacteria.

## Revendications

1. Procédé de détection d'une infection chez un mammifère par une bactérie acido-résistante du genre *Helicobacter*, dans lequel
(a) on incube un échantillon de selles du mammifère en utilisant (aa) un récepteur dans des conditions permettant une complexation d'un antigène de la bactérie acido-résistante avec le récepteur ; ou (ab) on incube deux récepteurs différents dans des conditions permettant une complexation d'un antigène de la bactérie acido-résistante avec les deux récepteurs, et dans lequel le récepteur selon (aa) ou les récepteurs selon (ab) lie(nt) de manière spécifique un antigène qui présente au moins chez une partie des mammifères, après passage intestinal, une structure correspondant à la structure native ou à la structure contre laquelle un mammifère produit des anticorps après infection ou immunisation avec la bactérie acido-résistante ou un extrait ou lysat de celle-ci ou une protéine en provenant ou un fragment de celle-ci ou un peptide synthétique; et
(b) on détecte la formation dudit un complexe antigène-récepteur selon (a)
dans lequel
ledit antigène est un antigène d'une catalase et
la détection de la formation dudit un complexe antigène-récepteur dans l'étape (b) est réalisée au moyen d'ELISA, RIA, Western Blot ou d'un procédé immunochromatographique.

2. Procédé selon la revendication 1, dans lequel la bactérie est une bactérie de l'espèce *Helicobacter pylori* ou *Helicobacter hepaticus.*

3. Procédé selon la revendication 2, dans lequel l'antigène est un antigène de H. pylori.

4. Procédé selon l'une quelconque des revendications 1 à 3, dans lequel le récepteur/les récepteurs est/sont un/des anticorps, un/des fragment(s) ou dérivé(s) de celui-ci/ceux-ci.

5. Procédé selon l'une quelconque des revendications 1 à 4, dans lequel on met en oeuvre, pour la détection, en outre un mélange de récepteurs, ledit mélange de récepteurs faisant fonction de capteur de l'antigène lorsque le récepteur est utilisé comme détecteur de l'antigène, et le mélange faisant fonction de détecteur de l'antigène lorsque le récepteur est utilisé comme capteur de l'antigène.

6. Procédé selon la revendication 5, dans lequel le mélange de récepteurs est un antisérum polyclonal.

7. Procédé selon la revendication 6, dans lequel l'antisérum polyclonal a été obtenu contre un lysat de la bactérie.

8. Procédé selon la revendication 7, dans lequel le lysat est un lysat avec de l'antigène enrichi.

9. Procédé selon la revendication 7 ou 8, dans lequel le lysat est un lysat avec de l'antigène immunodominant appauvri.

10. Procédé selon la revendication 8, dans lequel ledit antisérum polyclonal a été obtenu contre un antigène purifié ou un antigène fabriqué de manière (semi-)synthétique.

11. Procédé selon l'une quelconque des revendications 1 à 10, dans lequel le récepteur et/ou le mélangé de récepteurs lie(nt) un/des épitope(s) conformationnel(s).

12. Procédé selon l'une quelconque des revendications 4 à 11, dans lequel la chaîne lourde de l'anticorps liant un épitope de catalase présente au moins l'un des CDR suivants, et de préférence CDR3 :
| | |
|---|---|
| CDR1: | NYWIH |
| CDR2: | YINPATGSTSYNQDFQD |
| CDR3: | EGYDGFDS |

13. Procédé selon la revendication 12, dans lequel la chaîne lourde présente tous les trois CDR mentionnés.

14. Procédé selon la revendication 12 ou 13, dans lequel la séquence d'ADN codant pour la chaîne lourde de l'anticorps présente au moins l'un des CDR suivants, de préférence CDR3 et plus préférentiellement tous les trois CDR suivants :
| | |
|---|---|
| CDR1 : | AACTACTGGA TTCAC |
| CDR2 : | TACATTAATC CTGCCACTGG TTCCACTTCT TACAATCAGG ACTTTCAGGA C |
| CDR3 : | GAGGGGTACG ACGGGTTTGA CTCC |

15. Procédé selon l'une quelconque des revendications 4 à 11, dans lequel la chaîne légère de l'anticorps liant un épitope de catalase présente au moins l'un des CDR suivants, de préférence CDR3, et plus préférentiellement tous les trois CDR suivants :
| | |
|---|---|
| CDR1 : | SASSSVNYMY |
| CDR2: | DTSKLAS |
| CDR3: | QQWSSNPYT |

16. Procédé selon la revendication 15, dans lequel la séquence d'ADN codant pour la chaîne légère de l'anticorps présente au moins l'un des CDR suivants, de préférence CDR3 et plus préférentiellement tous les trois CDR suivants :
| | |
|---|---|
| CDR1 : | AGTGCCAGCT CAAGTGTAAA TTACATGTAC |
| CDR2 : | GACACATCCA AATTGGCTTC T |
| CDR3 : | CAGCAGTGGA GTAGTAATCC GTACACG |

17. Procédé selon l'une quelconque des revendications 4 à 11, dans lequel la chaîne lourde de l'anticorps liant un épitope de catalase présente au moins l'un des CDR suivants, de préférence CDR3, et plus préférentiellement tous les trois CDR suivants :
| | |
|---|---|
| CDR1 : | DTYVH |
| CDR2 : | KLDPANGKTKYDPIFQA |
| CDR3 : | PIYYASSWFAY |

18. Procédé selon la revendication 17, dans lequel la séquence d'ADN codant pour la chaîne lourde de l'anticorps présente au moins l'un des CDR suivants, de préférence CDR3 et plus préférentiellement tous les trois CDR suivants :
| | |
|---|---|
| CDR1: | GACACCTATGTGCAC |
| CDR2: | AAGATTGATCCTGCGAATGGTAAAACTAAATAT GACCCGATATTCCAGGCC |
| CDR3: | CCCATTTATTACGCTAGTTCCTGGTTTGCTTAC |

19. Procédé selon l'une quelconque des revendications 4 à 11, dans lequel la chaîne légère de l'anticorps liant un épitope de catalase présente au moins l'un des CDR suivants, de préférence CDR3, et plus préférentiellement tous les trois CDR suivants :
| | |
|---|---|
| CDR1 : | KASQDVGTSVA |
| CDR2 : | WTSTRHT |
| CDR3 : | QYSSSPT |

20. Procédé selon la revendication 19, dans lequel la séquence d'ADN codant pour la chaîne légère de l'anticorps présente au moins l'un des CDR suivants, de préférence CDR3 et plus préférentiellement tous les trois CDR suivants :
| | |
|---|---|
| CDR1 : | AAGGCCAGTCAGGATGTGGGTACTTCTGTTGCC |
| CDR2 : | TGGACATCCACCCGGCACACT |
| CDR3 : | CAGCAATATAGCAGCTCTCCCACG |

21. Procédé selon l'une quelconque des revendications 4 à 20, dans lequel les anticorps présentent dans les régions variables des chaînes légères et lourdes les séquences d'acides aminés représentées aux figures 1 et 2, 3 et 4.

22. Procédé selon l'une quelconque des revendications 4 à 21, dans lequel les domaines codants des régions variables des chaînes légères et lourdes présentent les séquences d'ADN représentées aux figures 1 et 2, 3 et 4.

23. Procédé selon l'une quelconque des revendications 1 à 22, dans lequel on réalise les étapes suivantes sur l'échantillon de selles avant l'incubation avec les anticorps :
(a) resuspendre ledit échantillon de selles dans un rapport compris entre 1 : 3 et 1 : 25, de préférence entre environ 1 : 5 et 1 : 10, plus préférentiellement de 1 : 5 dans un tampon de resuspension et
(b) mélanger sur un mélangeur Vortex.

24. Procédé selon l'une quelconque des revendications 1 à 23, dans lequel on utilise en RIA ou ELISA le même récepteur pour la liaison à la phase solide et pour la détection de l'épitope.

25. Procédé selon l'une quelconque des revendications 1 à 24, dans lequel le récepteur est fixé à un support.

26. Procédé selon l'une quelconque des revendications 1 à 25, dans lequel le récepteur est un anticorps monoclonal de souris.

27. Procédé selon l'une quelconque des revendications 1 à 26, dans lequel le procédé est un ELISA en une étape.

28. Procédé selon l'une quelconque des revendications 1 à 26, dans lequel le procédé est un ELISA en trois étapes.

29. Procédé selon la revendication 25, dans lequel le matériau support du support est un matériau support poreux.

30. Procédé selon la revendication 25 ou 29, dans lequel le matériau support est une bandelette pour tests.

31. Procédé selon la revendication 25, 29 ou 30, dans lequel le matériau support est en cellulose ou en un dérivé de cellulose.

32. Procédé selon l'une quelconque des revendications 1 à 31, dans lequel on utilise, pour la détection, de la plaque dentaire ou de la salive au lieu d'un échantillon de selles.

33. Procédé selon l'une quelconque des revendications 1 à 32, dans lequel il s'agit d'un procédé automatisé.

34. Procédé selon l'une quelconque des revendications 1 à 33, dans lequel le mammifère est un être humain.

35. Anticorps monoclonal, fragment ou dérivé de celui-ci, qui présente une région V qui comprend une combinaison des CDR représentés dans l'une quelconque des revendications 12 à 20.

36. Anticorps monoclonal, fragment ou dérivé de celui-ci, selon la revendication 35, qui présente l'une au moins des régions V représentées aux figures 1 et 2 ou 3 et 4.

37. Anticorps monoclonal, fragment ou dérivé de celui-ci, selon la revendication 35 ou 36, qui est un anticorps de souris ou un fragment ou dérivé de celui-ci ou un anticorps chimère, de préférence un anticorps humanisé ou un fragment ou dérivé de celui-ci.

38. Utilisation d'une composition de diagnostic comprenant un récepteur tel que défini à l'une quelconque des revendications 1 à 34 et 35 à 37, qui lie de manière spécifique un antigène d'une catalase d'*Helicobacter* et qui, le cas échéant, est fixé sur un matériau de support, pour la détection *d'Helicobacter* dans les selles.

39. Dispositif de test pour la détection d'au moins un épitope tel que défini dans l'une quelconque des revendications précédentes, comprenant :
(a) un récepteur tel que défini dans l'une quelconque des revendications 1 à 34, 35 à 37 qui lie de manière spécifique un antigène d'une catalase *d'Helicobacter,* fixé sur un matériau de support ; et
(b) un dispositif pour la préparation et l'analyse d'échantillons de selles.

40. Dispositif de test pour la détection d'au moins un épitope tel que défini dans l'une quelconque des revendications précédentes, comprenant :
(a) un récepteur tel que défini dans l'une quelconque des revendications 1 à 34, 35 à 37 qui lie de manière spécifique un antigène d'une catalase *d'Helicobacter,* le récepteur étant conjugué avec de l'or colloïdal, des particules de latex ou d'autres particules donnant de la couleur dont la taille se situe typiquement dans la gamme comprise entre 5 nm et 100 nm, de préférence entre 20 nm et 60 nm, d'une manière particulièrement préférée entre 40 nm et 60 nm (or) ou bien 200 nm et 500 nm (latex) ; et
(b) un dispositif pour la préparation et l'analyse d'échantillons de selles.

41. Trousse contenant
(a) un récepteur tel que défini dans l'une quelconque des revendications 1 à 34, 35 à 37 qui lie de manière spécifique un antigène d'une catalase *d'Helicobacter,* le cas échéant fixé sur un matériau de support ; et, le cas échéant, en outre
(b) un dispositif pour la préparation et l'analyse d'échantillons de selles.

42. Emballage contenant ledit dispositif de test selon la revendication 39 ou 40 ou ladite trousse selon la revendication 41.

43. Utilisation d'un seul récepteur qui lie de manière spécifique un épitope d'un antigène d'une bactérie acido-résistante appartenant au genre *Helicobacter,* ou de deux récepteurs qui lient de manière spécifique deux épitopes du même antigène, l'antigène étant de la catalase *d'Helicobacter,* pour la détection de l'infection par cette bactérie chez un mammifère dans les selles.
